# EUROPEAN PATENT APPLICATION

(11) **EP 2 343 280 A1**
(43) Date of publication of application: **13.07.2011**
(21) Application number: 09356065.4
(22) Date of filing: 10.12.2009
(51) Int. Cl.: C07D 215/20, C07D 215/38, A01N 43/42

(54) **Fungicide quinoline derivatives**

(71) Applicant: Bayer CropScience AG, 40789 Monheim (DE)
(72) Inventor: Bernier, David, 69004 Lyon (FR); Coqueron, Pierre-Yves, 69003 Lyon (FR); Es-Sayed, Mazen, 69270 Couzon au Mont d'Or (FR); Tuch, Arounarith, 69009 Lyon (FR)
(74) Representative: Guitton, Carole

(57) **Abstract**

The present invention relates to quinoline derivatives, their process of preparation, their use as fungicide active agents, particularly in the form of fungicide compositions, and methods for the control of phytopathogenic fungi, notably of plants, using these compounds or compositions.

## Description

### DESCRIPTION

The present invention relates to quinoline derivatives, their process of preparation, their use as fungicide active agents, particularly in the form of fungicide compositions, and to methods for the control of phytopathogenic fungi, notably of plants, using these compounds or compositions.

W02008/110355 and WO2009/030467 disclose quinoleine derivatives as fungicides. Nevertheless, they do not disclose compounds wherein the carbone at the alpha position of the amide is substituted by CN, CF3, F, CO2R, or heteroaryl, and said carbone is never quaternary. Additionally, the compounds disclosed in these documents do not prove to provide a comparable utility than the compounds according to the invention.

It is always of high-interest in agriculture to use novel pesticide compounds in order to avoid or to control the development of resistant strains to the active ingredients. It is also of high-interest to use novel compounds being more active than those already known, with the aim of decreasing the amounts of active compound to be used, whilst at the same time maintaining effectiveness at least equivalent to the already known compounds. We have now found a new family of compounds which possess the above mentioned effects or advantages.

Accordingly, the present invention provides a quinoline derivative of formula (I) 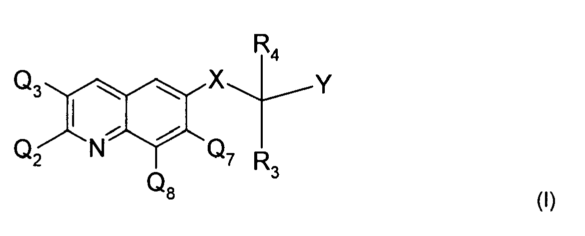 wherein
- Y is represented by 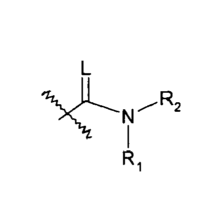 or 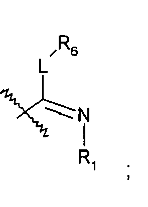
- X is O or S;
- L is O or S,
- Q2 is hydrogen, halogen, cyano, substituted or non-substituted, branched or non-branched C₁-C₈-alkyl, substituted or non-substituted C₃-C₆-cydoalkyl, C₃-C₁₂-(cycloalkyl)alkyl, substituted or non-substituted C₂-C₆-alkenyl, or substituted or non-substituted C₂-C₆-alkynyl,
- Q3 is halogen, or substituted or non-substituted C₂-C₆-alkynyl,
- Q7 is hydrogen, halogen, cyano, substituted or non-substituted, branched or non-branched C₁-C₈-alkyl, substituted or non-substituted C₁-C₆-haloalkyl, substituted or non-substituted C₃-C₆-cydoalkyl, C₃-C₁₂-(cycloalkyl)alkyl, substituted or non-substituted C₁-C₆-alkoxy, or alkylsulfinyl,
- Q8 is hydrogen, halogen, cyano, substituted or non-substituted, branched or non-branched C₁-C₈-alkyl, substituted or non-substituted C₁-C₆-haloalkyl, substituted or non-substituted C₃-C₆-cycloalkyl, C₃-C₁₂cycloalkyl)alkyl, substituted or non-substituted C₁-C₆-alkoxy, or alkylsulfinyl,
- R3 is halogen, cyano, substituted or non-substituted, branched or non-branched C₁-C₈-alkyl, substituted or non-substituted C₁-C₆-haloalkyl, substituted or non-substituted C₃-C₆-cycloalkyl, C₁-C₈-alkoxycarbonyl, or a heteroaryl non-substituted or substituted with substituted or non-substituted, branched or non-branched alkyl C₁-C₆ or cycloclalkyl C₃-C₆, wherein the heteroaryl is a 5-membered ring having one to three nitrogen atom(s) and is bound by a nitrogen atom, with the proviso that, when R4 is hydrogen, then R3 is not a C₁-C₈-alkyl,
- R4 is hydrogen, substituted or non-substituted, branched or non-branched C₁-C₈-alkyl, substituted or non-substituted C₁-C₈-alkoxy, C₁-C₄-alkylsulfinyl, S(O)nR7,
- R2 is hydrogen, substituted or non-substituted, branched or non-branched C₁-C₈-alkyl, substituted or non-substituted C₃-C₄-cycloalkyl, or substituted or non-substituted C₁-C₄-cyanoalkyl,
- R1 is heteroaryl, heteroarylalkyl, C₁-C₈-alkyloxyalkyl, C₃-C₅-cycloalkyl, C₁-C₈-alkyl, Alkynylalkyl, Alkylalkynylalkyl, Alkylalkynylcycloalkyl, or Alkoxylalkylalkynylalkyl,
- R6 is substituted or non-substituted, branched or non-branched C₁-C₈-alkyl, substituted or non-substituted C₁-C₆-haloalkyl, or substituted or non-substituted C₃-C₆-cycloalkyl,
- R7 is substituted or non-substituted, branched or non-branched C₁-C₈-alkyl,
- n is 0, 1 or 2.

Any of the compounds according to the invention can exist as one or more stereoisomers depending on the number of stereogenic units (as defined by the IUPAC rules) in the compound. The invention thus relates equally to all the stereoisomers, and to the mixtures of all the possible stereoisomers, in all proportions. The stereoisomers can be separated according to the methods which are known *per se* by the man ordinary skilled in the art.

According to the invention, the following generic terms are generally used with the following meanings:
- halogen means fluorine, chlorine, bromine or iodine ;
- heteroatom can be nitrogen, oxygen or sulphur ;
- unless indicated otherwise, a group or a substituent that is substituted according to the invention can be substituted by one or more of the following groups or atoms: a halogen atom, a nitro group, a hydroxy group, a cyano group, an amino group, a sulphenyl group, a pentafluoro-λ⁶-sulphenyl group, a formyl group, a substituted or non-substituted carbaldehyde O-(C₁C₈-alkyl)oxime, a formyloxy group, a formylamino group, a carbamoyl group, a N-hydroxycarbamoyl group, a (hydroxyimino)-C₁-C₆-alkyl group, a C₁-C₈-alkyl, a tri(C₁-C₈-alkyl)silyl-C₁-C₈-alkyl, C₁-C₈-cycloalkyl, tri(C₁-C₈-alkyl)silyl-C₁-C₈-Cycloalkyl, a C₁-C₈-halogenoalkyl having 1 to 5 halogen atoms, a C₁-C₈-halogenocycloalkyl having 1 to 5 halogen atoms, a C₂-C₈-alkenyl, a C₂-C₈-alkynyl, a C₂-C₈-alkenyloxy, a C₂-C₈-alkynyloxy, a C₁-C₈-alkylamino, a di-C₁-C₈-alkylamino, a C₁-C₈-alkoxy, a C₁-C₈-halogenoalkoxy having 1 to 5 halogen atoms, a C₁-C₈-alkylsulphenyl, a C₁-C₈-halogenoalkylsulphenyl having 1 to 5 halogen atoms, a C₂-C₈-alkenyloxy, a C₂-C₈-halogenoalkenyloxy having 1 to 5 halogen atoms, a C₃-C₈-alkynyloxy, a C₃-C₈-halogenoalkynyloxy having 1 to 5 halogen atoms, a C₁-C₈-alkylcarbonyl, a C₁-C₈-halogenoalkylcarbonyl having 1 to 5 halogen atoms, a C₁-C₈-alkylcarbamoyl, a di-C₁-C₈-alkylcarbamoyl, a N-C₁-C₈-alkyloxycarbamoyl, a C₁-C₈-alkoxycarbamoyl, a N-C₁-C₈-alkyl-C₁-C₈-alkoxycarbamoyl, a C₁-C₈-alkoxycarbonyl, a C₁-C₈-halogenoalkoxycarbonyl having 1 to 5 halogen atoms, a C₁-C₈-alkylcarbonyloxy, a C₁-C₈-halogenoalkylcarbonyloxy having 1 to 5 halogen atoms, a C₁-C₈-alkylcarbonylamino, a C₁-C₈-halogenoalkylcarbonylamino having 1 to 5 halogen atoms, substituted or non-substituted C₁-C₈-alkoxycarbonylamino, substituted or non-substituted C₁-C₈-halogenoalkoxycarbonylamino having 1 to 5 halogen atoms, a C₁-C₈-alkylaminocarbonyloxy, a di-C₁-C₈-alkylaminocarbonyloxy, a C₁-C₈-alkyloxycarbonyloxy, a C₁-C₈-alkylsulphenyl, a C₁-C₈-halogenoalkylsulphenyl having 1 to 5 halogen atoms, a C₁-C₈-alkylsulphinyl, a C₁-C₈-halogenoalkylsulphinyl having 1 to 5 halogen atoms, a C₁-C₈-alkylsulphonyl, a C₁-C₈-halogenoalkylsulphonyl having 1 to 5 halogen atoms, a C₁-C₈-alkylaminosulfamoyl, a di-C₁-C₈-alkylaminosulfamoyl, a (C₁-C₆-alkoxyiminO)-C₁-C₆-alkyl, a (C₁-C₆-alkenyloxyimino)-C₁-C₆-alkyl, a (C₁-C₆-alkynyloxyimino)-C₁-C₆-alkyl, (benzyloxyimino)-C₁-C₆-alkyl, C₁-C₈-alkoxyalkyl, C₁-C₈-halogenoalkoxyalkyl having 1 to 5 halogen atoms, benzyloxy, benzylsulphenyl; benzylamino, phenoxy, phenylsulphenyl, or phenylamino;
- the term "aryl" means phenyl or naphthyl.

Compounds according to the invention can be prepared according to the above described processes. It will nevertheless be understood that, on the basis of his general knowledge and of available publications, the skilled worker will be able to adapt these processes according to the specifics of each of the compounds according to the invention that is desired to be synthesised.

In a further aspect, the present invention also relates to a fungicide composition comprising an effective and non-phytotoxic amount of an active compound of formula (I).

The expression "effective and non-phytotoxic amount" means an amount of composition according to the invention that is sufficient to control or destroy the fungi present or liable to appear on the crop sand that does not entail any appreciable symptom of phytotoxicity for the said crops. Such an amount can vary within a wide range depending on the fungus to be controlled, the type of crop, the climatic conditions and the compounds included in the fungicide composition according to the invention. This amount can be determined by systematic field trials, that are within the capabilities of a person skilled in the art.

Thus, according to the invention, there is provided a fungicide composition comprising, as an active ingredient, an effective amount of a compound of formula (I) as herein-defined and an agriculturally acceptable support, carrier or filler.

According to the invention, the term "support" denotes a natural or synthetic, organic or inorganic compound with that the active compound of formula (I) is combined or associated to make it easier to apply, notably to the parts of the plant. This support is thus generally inert and should be agriculturally acceptable. The support can be a solid or a liquid. Examples of suitable supports include clays, natural or synthetic silicates, silica, resins, waxes, solid fertilisers, water, alcohols, in particular butanol, organic solvents, mineral and plant oils and derivatives thereof. Mixtures of such supports can also be used.

The composition according to the invention can also comprise additional components. In particular, the composition can further comprise a surfactant. The surfactant can be an emulsifier, a dispersing agent or a wetting agent of ionic or non-ionic type or a mixture of such surfactants. Mention can be made, for example, of polyacrylic acid salts, lignosulphonic acid salts, phenolsulphonic or naphthalenesulphonic acid salts, polycondensates of ethylene oxide with fatty alcohols or with fatty acids or with fatty amines, substituted phenols (in particular alkylphenols or arylphenols), salts of sulphosuccinic acid esters, taurine derivatives (in particular alkyl taurates), phosphoric esters of polyoxyethylated alcohols or phenols, fatty acid esters of polyolsand derivatives of the above compounds containing sulphate, sulphonate and phosphate functions. The presence of at least one surfactant is generally essential when the active compound and/or the inert support are water-insoluble and when the vector agent for the application is water. Preferably, surfactant content can be comprised from 5% to 40% by weight of the composition.

Optionally, additional components can also be included, e.g. protective colloids, adhesives, thickeners, thixotropic agents, penetration agents, stabilisers, sequestering agents. More generally, the active compounds can be combined with any solid or liquid additive, that complies with the usual formulation techniques.

In general, the composition according to the invention can contain from 0.05 to 99% by weight of active compound, preferably 10 to 70% by weight.

Compositions according to the invention can be used in various forms such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure),gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra low volume (ULV) liquid, ultra low volume (ULV) suspension, water dispersible granules or tablets, water dispersible powder for slurry treatment, water soluble granules or tablets, water soluble powder for seed treatment and wettable powder. These compositions include not only compositions that are ready to be applied to the plant or seed to be treated by means of a suitable device, such as a spraying or dusting device, but also concentrated commercial compositions that must be diluted before application to the crop.

The compounds according to the invention can also be mixed with one or more insecticide, fungicide, bactericide, attractant, acaricide or pheromone active substance or other compounds with biological activity. The mixtures thus obtained have normally a broadened spectrum of activity. The mixtures with other fungicide compounds are particularly advantageous.

Examples of suitable fungicide mixing partners can be selected in the following lists:
B1) a compound capable to inhibit the nucleic acid synthesis like benalaxyl, benalaxyl-M, bupirimate, clozylacon, dimethirimol, ethirimol, furalaxyl, hymexazol, mefenoxam, metalaxyl, metalaxyl-M, ofurace, oxadixyl, oxolinic acid ;
B2) a compound capable to inhibit the mitosis and cell division like benomyl, carbendazim, diethofencarb, ethaboxam, fuberidazole, pencycuron, thiabendazole, thiophanate-methyl, zoxamide;
B3) a compound capable to inhibit the respiration for example
   as Cl-respiration inhibitor like diflumetorim ;
   as Cll-respiration inhibitor like boscalid, carboxin, fenfuram, flutolanil, furametpyr, furmecyclox, mepronil, oxycarboxin, penthiopyrad, thifluzamide ;
   as Clll-respiration inhibitor like amisulbrom, azoxystrobin, cyazofamid, dimoxystrobin, enestrobin, famoxadone, fenamidone, fluoxastrobin, kresoxim-methyl, metominostrobin, orysastrobin, picoxystrobin, pyraclostrobin, trifloxystrobin ;
B4) a compound capable of to act as an uncoupler like dinocap, fluazinam, meptyldinocap ;
B5) a compound capable to inhibit ATP production like fentin acetate, fentin chloride, fentin hydroxide, silthiofam ;
B6) a compound capable to inhibit AA and protein biosynthesis like andoprim, blasticidin-S, cyprodinil, kasugamycin, kasugamycin hydrochloride hydrate, mepanipyrim, pyrimethanil ;
B7) a compound capable to inhibit the signal transduction like fenpiclonil, fludioxonil, quinoxyfen ;
B8) a compound capable to inhibit lipid and membrane synthesis like biphenyl, chlozolinate, edifenphos, etridiazole, iodocarb, iprobenfos, iprodione, isoprothiolane, procymidone, propamocarb, propamocarb hydrochloride, pyrazophos, tolclofos-methyl, vinclozolin ;
B9) a compound capable to inhibit ergosterol biosynthesis like aldimorph, azaconazole, bitertanol, bromuconazole, cyproconazole, diclobutrazole, difenoconazole, diniconazole, diniconazole-M, dodemorph, dodemorph acetate, epoxiconazole, etaconazole, fenarimol, fenbuconazole, fenhexamid, fenpropidin, fenpropimorph, fluquinconazole, flurprimidol, flusilazole, flutriafol, furconazole, furconazolecis, hexaconazole, imazalil, imazalil sulfate, imibenconazole, ipconazole, metconazole, myclobutanil, naftifine, nuarimol, oxpoconazole, paclobutrazol, pefurazoate, penconazole, prochloraz, propiconazole, prothioconazole, pyributicarb, pyrifenox, simeconazole, spiroxamine, tebuconazole, terbinafine, tetraconazole, triadimefon, triadimenol, tridemorph, triflumizole, triforine, triticonazole, uniconazole, viniconazole, voriconazole ;
B10) a compound capable to inhibit cell wall synthesis like benthiavalicarb, dimethomorph, flumorph, iprovalicarb, mandipropamid, polyoxins, polyoxorim, validamycin A ;
B11) a compound capable to inhibit melanine biosynthesis like carpropamid, diclocymet, fenoxanil, phthalide, pyroquilon, tricyclazole ;
B12) a compound capable to induce a host defence like acibenzolar-S-methyl, probenazole, tiadinil ;
B13) a compound capable to have a multisite action like Bordeaux mixture, captafol, captan, chlorothalonil, copper naphthenate, copper oxide, copper oxychloride, copper preparations such as copper hydroxide, copper sulphate, dichlofluanid, dithianon, dodine, dodine free base, ferbam, fluorofolpet, folpet, guazatine, guazatine acetate, iminoctadine, iminoctadine albesilate, iminoctadine triacetate, mancopper, mancozeb, maneb, metiram, metiram zinc, oxine-copper, propineb, sulphur and sulphur preparations including calcium polysulphide, thiram, tolylfluanid, zineb, ziram ;
B14) a compound selected in the following list: 2,3-dibutyl-6-chlorothieno[2,3-d]pyrimidin-4(3H)-one, ethyl (2Z)-3-amino-2-cyano-3-phenylprop-2-enoate, N-[2-(1,3-dimethylbutyl)phenyl]-5-fluoro-1,3-dimethyl-1H-pyrazole-4-carboxamide, N-{2-[1,1'-bi(cyclopropyl)-2-yl]phenyl}-3-(difluoromethyl)-1-methyl-1H-pyrazole-4-carboxamide, 3-(difluoromethyl)-1-methyl-N-(3',4',5'-trifluorobiphenyl-2-yl)-1H-pyrazole-4-carboxamide, 3-(difluoromethyl)-N-[4-fluoro-2-(1,1,2,3,3,3-hexafluoropropoxy)phenyl]-1-methyl-1H-pyrazole-4-carboxamide, (2E)-2-(2-{[6-(3-chloro-2-methylphenoxy)-5-fluoropyrimidin-4-yl]oxy}phenyl)-2-(methoxyimino)-N-methylethanamide, (2E)-2-{2-[({[(2E,3E)-4-(2,6-dichlorophenyl)but-3-en-2-ylidene]amino}oxy)methyl]phenyl}-2-(methoxyimino)-N-methylethanamide, 2-chloro-N-(1,1,3-trimethyl-2,3-dihydro-1H-inden-4-yl)pyridine-3-carboxamide, N-(3-ethyl-3,5,5-trimethylcyclohexyl)-3-(formylamino)-2-hydroxy benzamide, 5-methoxy-2-methyl-4-(2-{[({(1E)-1-[3-(trlfluoromethyl)phenyl]ethylidene}amino)oxy] methyl}phenyl)-2,4-dihydro-3H-1,2,4-triazol-3-one, (2E)-2-(methoxyimino)-N-methyl-2-(2-{[({(1E)-1-[3-(trifluoromethyl)phenyl]ethylidene)amino)oxy] methyl}phenyl)ethanamide, (2E)-2-(methoxyimino)-N-methyl-2-{2-[(E)-({1-[3-(trifluoromethyl)phenyl]ethoxy}imino)methyl]phenyl} ethanamide, (2E)-2-{2-[({[(1E)-1-(3-{[(E)-1-fluoro-2-phenylethenyl]oxy}phenyl)ethylidene]amino} oxy)methyl]phenyl}-2-(methoxyimino)-N-methylethanamide, 1-(4-chlorophenyl)-2-(1H-1,2,4-triazol-1-yl)cycloheptanol, methyl 1-(2,2-dimethy)-2,3-dihydro-1H-inden-1-yl)-1H-imidazole-5-carboxylate, N-ethyl-N-methyl-N'-{2-methyl-5-(trifluoromethyl)-4-[3-(trimethylsilyl)propoxy] phenyl}imidoformamide, N'-{5-(difluoromethyl)-2-methyl-4-(3-(trimethylsilyl)propoxy]phenyl}-N-ethyl-N-methylimidoformamide, O-{1-[(4-methoxyphenoxy)methyl]-2,2-dimethylpropyl} 1H-imidazole-1-carbothioate, N-[2-(4-{[3-(4-chlorophenyl)prop-2-yn-1-yl]oxy}-3-methoxyphenyl) ethyl]-N²-(methylsulfonyl)valinamide, 5-chloro-7-(4-methylpiperidin-1-yl)-6-(2,4,6-trifluorophenyl) [1,2,4]triazolo[1,5-a]pyrimidine, 5-amino-1,3,4-thiadiazole-2-thiol, propamocarb-fosetyl, 1-[(4-methoxyphenoxy)methyl]-2,2-dimethylpropyl 1H-imidazole-1-carboxylate, 1-methyl-N-[2-(1,1,2,2-tetrafluoroethoxy)phenyl]-3-(trifluoromethyl)-1H-pyrazole-4-carboxamide, 2,3,5,6-tetrachloro-4-(methylsulfonyl)pyridine, 2-butoxy-6-iodo-3-propyl-4H-chromen-4-one, 2-phenylphenol and salts, 3-(difluoromethyl)-1-methyl-N-[2-(1,1,2,2-tetrafluoroethoxy)phenyl]-1H-pyrazole-4-carboxamide, 3,4,5-trichloropyridine-2,6-dicarbonitrile, 3-[5-(4-chlorophenyl)-2,3-dimethylisoxazolidin-3-yl]pyridine, 3-chloro-5-(4-chlorophenyl)-4-(2,6-difluorophenyl)-6-methylpyridazine, 4-(4-chlorophenyl)-5-(2,6-difluorophenyl)-3,6-dimethylpyridazine, quinolin-8-ol, quinolin-8-ol sulfate (2:1) (salt), 5-methyl-6-octyl-3,7-dihydro[1,2,4]triazolo[1,5-a]pyrimidin-7-amine, 5-ethyl-6-octyl-3,7-dihydro[1,2,4]triazolo[1,5-a]pyrimidin-7-amine, benthiazole, bethoxazin, capsimycin, carvone, chinomethionat, chloroneb, cufraneb, cyflufenamid, cymoxanil, cyprosulfamide, dazomet, debacarb, dichlorophen, diclomezine, dicloran, difenzoquat, difenzoquat methylsulphate, diphenylamine, ecomate, ferimzone, flumetover, fluopicolide, fluoroimide, flusulfamide, fosetyl-aluminium, fosetyl-calcium, fosetyl-sodium, hexachlorobenzene, irumamycin, isotianil, methasulfocarb, methyl (2E)-2-{2-[({cyclopropyl[(4-methoxyphenyl)imino]methyl}thio)methyl]phenyl}-3-methoxyacrylate, methyl isothiocyanate, metrafenone, (5-bromo-2-methoxy-4-methylpyridin-3-yl)(2,3,4-trimethoxy-6-methylphenyl) methanone, mildiomycin, toinifanide, N-(4-chlorobenzyl)-3-[3-methoxy-4-(prop-2-yn-1-yloxy)phenyl]propanamide, N-[(4-chlorophenyl)(cyano)methyl]-3-[3-methoxy-4-(prop-2-yn-1-yloxy)phenyl]propanamide, N-[(5-bromo-3-chloropyridin-2-yl)methyl]-2,4-dichloropyridine-3-carboxamide, N-[1-(5-bromo-3-chloropyridin-2-yl)ethyl]-2,4-dichloropyridine-3-carboxamide, N-[1-(5-bromo-3-chloropyridin-2-yl)ethyl]-2-fluoro-4-iodopyridine-3-carboxamide, N-{(Z)-[(cyclopropylmethoxy)imino][6-(difluoromethoxy)-2,3-difluorophenyl]methyl}-2-phenylacetamide, N-{(E)-[(cyclopropylmethoxy)imino][6-(difluoromethoxy)-2,3-difluorophenyl]methyl}-2-phenylacetamide, natamycin, nickel dimethyldithiocarbamate, nitrothal-isopropyl, octhilinone, oxamocarb, oxyfenthiin, pentachlorophenol and salts, phenazine-1-carboxylic acid, phenothrin, phosphorous acid and its salts, propamocarb fosetylate, propanosine-sodium, proquinazid, pyrrolnitrine, quintozene, S-prop-2-en-1-yl 5-amino-2-(1-methylethyl)-4-(2-methylphenyl)-3-oxo-2,3-dihydro-1H-pyrazole-1-carbothioate, tecloftalam, tecnazene, triazoxide, trichlamide, 5-chloro-N'-phenyl-N'-prop-2-yn-1-ylthiophene-2-sulfonohydrazide and zarilamid.

The composition according to the invention comprising a mixture of a compound of formula (I) with a bactericide compound can also be particularly advantageous. Examples of suitable bactericide mixing partners can be selected in the following list: bronopol, dichlorophen, nitrapyrin, nickel dimethyldithiocarbamate, kasugamycin, octhilinone, furancarboxylic acid, oxytetracycline, probenazole, streptomycin, tecloftalam, copper sulphate and other copper preparations.

The compounds of formula (I) and the fungicide composition according to the invention can be used to curatively or preventively control the phytopathogenic fungi of plants or crops.

Thus, according to a further aspect of the invention, there is provided a method for curatively or preventively controlling the phytopathogenic fungi of plants or crops **characterised in that** a compound of formula (I) or a fungicide composition according to the invention is applied to the seed, the plant or to the fruit of the plant or to the soil wherein the plant is growing or wherein it is desired to grow.

The method of treatment according to the invention can also be useful to treat propagation material such as tubers or rhizomes, but also seeds, seedlings or seedlings pricking out and plants or plants pricking out. This method of treatment can also be useful to treat roots. The method of treatment according to the invention can also be useful to treat the overground parts of the plant such as trunks, stems or stalks, leaves, flowers and fruit of the concerned plant.

Among the plants that can be protected by the method according to the invention, mention can be made of cotton; flax; vine; fruit or vegetable crops such as *Rosaceae sp.* (for instance pip fruit such as apples and pears, but also stone fruit such as apricots, almonds and peaches), *Ribesioidae sp., Juglandaceae sp., Betulaceae sp.,* Anacardiaceae *sp*., Fagaceae *sp*., Moraceae *sp*., Oleaceae *sp*., *Actinidaceae sp., Lauraceae sp*., *Musaceae sp*. (for instance banana trees and plantins), *Rubiaceae sp*., *Theaceae sp*., *Sterculiceae sp., Rutaceae sp.* (for instance lemons oranges and grapefruit); *Solanaceae* sp. (for instance tomatoes), *Liliaceae sp., Asteraceae sp.* (for instance lettuces), *Umbelliferae sp., Cruciferae sp., Chenopodiaceae sp., Cucurbitaceae sp., Papilionaceae sp.* (for instance peas), *Rosaceae* sp. (for instance strawberries); major crops such as *Graminae sp.* (for instance maize, lawn or cereals such as wheat, rye, rice, barley and triticale), *Asteraceae sp*. (for instance sunflower), *Cruciferae sp.* (for instance colza), *Fabacae sp.* (for instance peanuts), *Papilionaceae sp.* (for instance soybean), *Solanaceae* sp. (for instance potatoes), *Chenopodiaceae sp.* (for instance beetroots), *Elaeis sp.* (for instance oil palm); horticultural and forest crops; as well as genetically modified homologues of these crops.

The method of treatment according to the invention can be used in the treatment of genetically modified organisms (GMOs), e.g. plants or seeds. Genetically modified plants (or transgenic plants) are plants in which a heterologous gene has been stably integrated into the genome. The expression "heterologous gene" essentially means a gene which is provided or assembled outside the plant and when introduced in the nuclear, chloroplastic or mitochondrial genome gives the transformed plant new or improved agronomic or other properties by expressing a protein or polypeptide of interest or by downregulating or silencing other gene(s) which are present in the plant (using for example, antisense technology, co suppression technology or RNA interference - RNAi - technology). A heterologous gene that is located in the genome is also called a transgene. A transgene that is defined by its particular location in the plant genome is called a transformation or transgenic event.

Depending on the plant species or plant cultivars, their location and growth conditions (soils, climate, vegetation period, diet), the treatment according to the invention may also result in superadditive ("synergistic") effects. Thus, for example, reduced application rates and/or a widening of the activity spectrum and/or an increase in the activity of the active compounds and compositions which can be used according to the invention, better plant growth, increased tolerance to high or low temperatures, increased tolerance to drought or to water or soil salt content, increased flowering performance, easier harvesting, accelerated maturation, higher harvest yields, bigger fruits, larger plant height, greener leaf color, earlier flowering, higher quality and/or a higher nutritional value of the harvested products, higher sugar concentration within the fruits, better storage stability and/or processability of the harvested products are possible, which exceed the effects which were actually to be expected.

At certain application rates, the active compound combinations according to the invention may also have a strengthening effect in plants. Accordingly, they are also suitable for mobilizing the defense system of the plant against attack by unwanted phytopathogenic fungi and/ or microorganisms and/or viruses. This may, if appropriate, be one of the reasons of the enhanced activity of the combinations according to the invention, for example against fungi. Plant-strengthening (resistance-inducing) substances are to be understood as meaning, in the present context, those substances or combinations of substances which are capable of stimulating the defense system of plants in such a way that, when subsequently inoculated with unwanted phytopathogenic fungi and/ or microorganisms and/or viruses, the treated plants display a substantial degree of resistance to these unwanted phytopathogenic fungi and/ or microorganisms and/or viruses. In the present case, unwanted phytopathogenic fungi and/ or microorganisms and/or viruses are to be understood as meaning phytopathogenic fungi, bacteria and viruses. Thus, the substances according to the invention can be employed for protecting plants against attack by the abovementioned pathogens within a certain period of time after the treatment. The period of time within which protection is effected generally extends from 1 to 10 days, preferably 1 to 7 days, after the treatment of the plants with the active compounds.

Plants and plant cultivars which are preferably to be treated according to the invention include all plants which have genetic material which impart particularly advantageous, useful traits to these plants (whether obtained by breeding and/or biotechnological means).

Plants and plant cultivars which are also preferably to be treated according to the invention are resistant against one or more biotic stresses, i.e. said plants show a better defense against animal and microbial pests, such as against nematodes, insects, mites, phytopathogenic fungi, bacteria, viruses and/or viroids.

Plants and plant cultivars which may also be treated according to the invention are those plants which are resistant to one or more abiotic stresses. Abiotic stress conditions may include, for example, drought, cold temperature exposure, heat exposure, osmotic stress, flooding, increased soil salinity, increased mineral exposure, ozon exposure, high light exposure, limited availability of nitrogen nutrients, limited availability of phosphorus nutrients, shade avoidance.

Plants and plant cultivars which may also be treated according to the invention, are those plants characterized by enhanced yield characteristics. Increased yield in said plants can be the result of, for example, improved plant physiology, growth and development, such as water use efficiency, water retention efficiency, improved nitrogen use, enhanced carbon assimilation, improved photosynthesis, increased germination efficiency and accelerated maturation. Yield can furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content, protein content, oil content and composition, nutritional value, reduction in anti-nutritional compounds, improved processability and better storage stability.

Plants that may be treated according to the invention are hybrid plants that already express the characteristic of heterosis or hybrid vigor which results in generally higher yield, vigor, health and resistance towards biotic and abiotic stress factors. Such plants are typically made by crossing an inbred male-sterile parent line (the female parent) with another inbred male-fertile parent line (the male parent). Hybrid seed is typically harvested from the male sterile plants and sold to growers. Male sterile plants can sometimes (e.g. in corn) be produced by detasseling, i.e. the mechanical removal of the male reproductive organs (or males flowers) but, more typically, male sterility is the result of genetic determinants in the plant genome. In that case, and especially when seed is the desired product to be harvested from the hybrid plants it is typically useful to ensure that male fertility in the hybrid plants is fully restored. This can be accomplished by ensuring that the male parents have appropriate fertility restorer genes which are capable of restoring the male fertility in hybrid plants that contain the genetic determinants responsible for male-sterility. Genetic determinants for male sterility may be located in the cytoplasm. Examples of cytoplasmic male sterility (CMS) were for instance described in Brassica species (W0 1992/005251, WO 1995/009910, WO 1998/27806, WO 2005/002324, WO 2006/021972 and US 6,229,072). However, genetic determinants for male sterility can also be located in the nuclear genome. Male sterile plants can also be obtained by plant biotechnology methods such as genetic engineering. A particularly useful means of obtaining male-sterile plants is described in WO 1989/10396 in which, for example, a ribonuclease such as barnase is selectively expressed in the tapetum cells in the stamens. Fertility can then be restored by expression in the tapetum cells of a ribonuclease inhibitor such as barstar (e.g. WO 1991/002069).

Plants or plant cultivars (obtained by plant biotechnology methods such as genetic engineering) which may be treated according to the invention are herbicide-tolerant plants, i.e. plants made tolerant to one or more given herbicides. Such plants can be obtained either by genetic transformation, or by selection of plants containing a mutation imparting such herbicide tolerance.

Herbicide-tolerant plants are for example glyphosate-tolerant plants, i.e. plants made tolerant to the herbicide glyphosate or salts thereof. Plants can be made tolerant to glyphosate through different means. For example, glyphosate-tolerant plants can be obtained by transforming the plant with a gene encoding the enzyme 5-enolpyruvylshikimate-3-phosphate synthase (EPSPS). Examples of such EPSPS genes are the AroA gene (mutant CT7) of the bacterium *Salmonella typhimurium* (Comai et al., Science (1983), 221, 370-371), the CP4 gene of the bacterium *Agrobacterium sp.* (Barry et al., Curr. Topics Plant Physiol. (1992), 7, 139-145), the genes encoding a Petunia EPSPS (Shah et al., Science (1986), 233, 478-481), a Tomato EPSPS (Gasser et al., J. Biol. Chem. (1988),263, 4280-4289), or an Eleusine EPSPS (WO 2001/66704). It can also be a mutated EPSPS as described in for example EP-A 0837944, WO 2000/066746, WO 2000/066747 or WO 2002/026995. Glyphosate-tolerant plants can also be obtained by expressing a gene that encodes a glyphosate oxido-reductase enzyme as described in US 5,776,760 and US 5,463,175. Glyphosate-tolerant plants can also be obtained by expressing a gene that encodes a glyphosate acetyl transferase enzyme as described in for example WO 2002/036782, WO 2003/092360, WO 2005/012515 and WO 2007/024782. Glyphosate-tolerant plants can also be obtained by selecting plants containing naturally-occurring mutations of the above-mentioned genes, as described in for example WO 2001/024615 or WO 2003/013226.

Other herbicide resistant plants are for example plants that are made tolerant to herbicides inhibiting the enzyme glutamine synthase, such as bialaphos, phosphinothricin or glufosinate. Such plants can be obtained by expressing an enzyme detoxifying the herbicide or a mutant glutamine synthase enzyme that is resistant to inhibition. One such efficient detoxifying enzyme is an enzyme encoding a phosphinothricin acetyltransferase (such as the bar or pat protein from Streptomyces species). Plants expressing an exogenous phosphinothricin acetyltransferase are for example described in US 5,561,236; US 5,648,477; US 5,646,024; US 5,273,894; US 5,637,489; US 5,276,268; US 5,739,082; US 5,908,810 and US 7,112,665.

Further herbicide-tolerant plants are also plants that are made tolerant to the herbicides inhibiting the enzyme hydroxyphenylpyruvatedioxygenase (HPPD). Hydroxyphenylpyruvatedioxygenases are enzymes that catalyze the reaction in which para-hydroxyphenylpyruvate (HPP) is transformed into homogentisate. Plants tolerant to HPPD-inhibitors can be transformed with a gene encoding a naturally-occurring resistant HPPD enzyme, or a gene encoding a mutated HPPD enzyme as described in WO 1996/038567, WO 1999/024585 and WO 1999/024586. Tolerance to HPPD-inhibitors can also be obtained by transforming plants with genes encoding certain enzymes enabling the formation of homogentisate despite the inhibition of the native HPPD enzyme by the HPPD-inhibitor. Such plants and genes are described in WO 1999/034008 and WO 2002/36787. Tolerance of plants to HPPD inhibitors can also be improved by transforming plants with a gene encoding an enzyme prephenate dehydrogenase in addition to a gene encoding an HPPD-tolerant enzyme, as described in WO 2004/024928.

Still further herbicide resistant plants are plants that are made tolerant to acetolactate synthase (ALS) inhibitors. Known ALS-inhibitors include, for example, sulfonylurea, imidazolinone, triazolopyrimidines, pyrimidinyloxy(thio)benzoates, and/or sulfonylaminocarbonyltriazolinone herbicides. Different mutations in the ALS enzyme (also known as acetohydroxyacid synthase, AHAS) are known to confer tolerance to different herbicides and groups of herbicides, as described for example in Tranel and Wright, Weed Science (2002), 50, 700-712, but also, in US 5,605,011, US 5,378,824, US 5,141,870, and US 5,013,659. The production of sulfonylurea-tolerant plants and imidazolinone-tolerant plants is described in US 5,605,011; US 5,013,659; US 5,141,870; US 5,767,361; US 5,731,180; US 5,304,732; US 4,761,373; US 5,331,107; US 5,928,937; and US 5,378,824; and international publication WO 1996/033270. Other imidazolinone-tolerant plants are also described in for example WO 2004/040012, WO 2004/106529, WO 2005/020673, WO 2005/093093, WO 2006/007373, WO 2006/015376, WO 2006/024351, and WO 2006/060634. Further sulfonylurea- and imidazolinone-tolerant plants are also described in for example WO 2007/024782.

Other plants tolerant to imidazolinone and/or sulfonylurea can be obtained by induced mutagenesis, selection in cell cultures in the presence of the herbicide or mutation breeding as described for example for soybeans in US 5,084,082, for rice in WO 1997/41218, for sugar beet in US 5,773,702 and WO 1999/057965 , for lettuce in US 5,198,599, or for sunflower in WO 2001/065922.

Plants or plant cultivars (obtained by plant biotechnology methods such as genetic engineering) which may also be treated according to the invention are insect-resistant transgenic plants, i.e. plants made resistant to attack by certain target insects. Such plants can be obtained by genetic transformation, or by selection of plants containing a mutation imparting such insect resistance.

An "insect-resistant transgenic plant", as used herein, includes any plant containing at least one transgene comprising a coding sequence encoding:
1) an insecticidal crystal protein from *Bacillus thuringiensis* or an insecticidal portion thereof, such as the insecticidal crystal proteins listed by Crickmore et al., Microbiology and Molecular Biology Reviews (1998), 62, 807-813, updated by Crickmore et al. (2005) at the *Bacillus thuringiensis* toxin nomenclature, online at:
   http://www.lifesci.sussex.ac.uk/Home/Neil_Crickmore/Bt/), or insecticidal portions thereof, e.g., proteins of the Cry protein classes Cry1Ab, Cry1Ac, Cry1F, Cry2Ab, Cry3Aa, or Cry3Bb or insecticidal portions thereof; or
2) a crystal protein from *Bacillus thuringiensis* or a portion thereof which is insecticidal in the presence of a second other crystal protein from *Bacillus thuringiensis* or a portion thereof, such as the binary toxin made up of the Cry34 and Cry35 crystal proteins (Moellenbeck et al., Nat. Biotechnol. (2001), 19, 668-72; Schnepf et al., Applied Environm. Microbiol. (2006), 71, 1765-1774); or
3) a hybrid insecticidal protein comprising parts of different insecticidal crystal proteins from *Bacillus thuringiensis,* such as a hybrid of the proteins of 1) above or a hybrid of the proteins of 2) above, e.g., the Cry1A.105 protein produced by corn event MON98034 (WO 2007/027777); or
4) a protein of any one of 1) to 3) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation, such as the Cry3Bb1 protein in corn events MON863 or MON88017, or the Cry3A protein in corn event MIR604;
5) an insecticidal secreted protein from *Bacillus thuringiensis* or *Bacillus cereus,* or an insecticidal portion thereof, such as the vegetative insecticidal (VIP) proteins listed at:
   http://www.lifesci.sussex.ac.uk/home/Neil_Crickmore/Bt/vip.html, e.g., proteins from the VIP3Aa protein class; or
6) a secreted protein from *Bacillus thuringiensis* or *Bacillus cereus* which is insecticidal in the presence of a second secreted protein from *Bacillus thuringiensis* or *B. cereus,* such as the binary toxin made up of the VIP1A and VIP2A proteins (WO 1994/21795); or
7) a hybrid insecticidal protein comprising parts from different secreted proteins from *Bacillus thuringiensis* or *Bacillus cereus,* such as a hybrid of the proteins in 1) above or a hybrid of the proteins in 2) above; or
8) a protein of any one of 1) to 3) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein), such as the VIP3Aa protein in cotton event COT102.

Of course, an insect-resistant transgenic plant, as used herein, also includes any plant comprising a combination of genes encoding the proteins of any one of the above classes 1 to 8. In one embodiment, an insect-resistant plant contains more than one transgene encoding a protein of any one of the above classes 1 to 8, to expand the range of target insect species affected when using different proteins directed at different target insect species, or to delay insect resistance development to the plants by using different proteins insecticidal to the same target insect species but having a different mode of action, such as binding to different receptor binding sites in the insect.

Plants or plant cultivars (obtained by plant biotechnology methods such as genetic engineering) which may also be treated according to the invention are tolerant to abiotic stresses. Such plants can be obtained by genetic transformation, or by selection of plants containing a mutation imparting such stress resistance. Particularly useful stress tolerance plants include:
a. plants which contain a transgene capable of reducing the expression and/or the activity of poly(ADP-ribose)polymerase (PARP) gene in the plant cells or plants as described in WO 2000/004173 or WO2006/045633 or PCT/EP07/004142.
b. plants which contain a stress tolerance enhancing transgene capable of reducing the expression and/or the activity of the PARG encoding genes of the plants or plants cells, as described e.g. in WO 2004/090140.
c. plants which contain a stress tolerance enhancing transgene coding for a plant- functional enzyme of the nicotinamide adenine dinucleotide salvage synthesis pathway including nicotinamidase, nicotinate phosphoribosyltransferase, nicotinic acid mononucleotide adenyl transferase, nicotinamide adenine dinucleotide synthetase or nicotine amide phosphoribosyltransferase as described e.g. in WO2006/032469 or WO 2006/133827 or PCT/EP07/002433.

Plants or plant cultivars (obtained by plant biotechnology methods such as genetic engineering) which may also be treated according to the invention show altered quantity, quality and/or storage-stability of the harvested product and/or altered properties of specific ingredients of the harvested product such as :
1) transgenic plants which synthesize a modified starch, which in its physical-chemical characteristics, in particular the amylose content or the amylose/amylopectin ratio, the degree of branching, the average chain length, the side chain distribution, the viscosity behaviour, the gelling strength, the starch grain size and/or the starch grain morphology, is changed in comparison with the synthesised starch in wild type plant cells or plants, so that this is better suited for special applications. Said transgenic plants synthesizing a modified starch are disclosed, for example, in EP 0571427, WO 1995/004826, EP 0719338, WO 1996/15248, WO 1996/19581, WO 1996/27674, WO 1997/11188, WO 1997/26362, WO 1997/32985, WO 1997/42328, WO 1997/44472, WO 1997/45545, WO 1998/27212, WO 1998/40503, WO99/58688, WO 1999/58690, WO 1999/58654, WO 2000/008184, WO 2000/008185, WO 2000/008175, WO 2000/28052, WO 2000/77229, WO 2001/12782, WO 2001/12826, WO 2002/101059, WO 2003/071860, WO 2004/056999, WO 2005/030942, WO 2005/030941, WO 2005/095632, WO 2005/095617, WO 2005/095619, WO 2005/095618, WO 2005/123927, WO 2006/018319, WO 2006/103107, WO 2006/108702, WO 2007/009823, WO 2000/22140, WO 2006/063862, WO 2006/072603, WO 2002/034923, EP 06090134.5, EP 06090228.5, EP 06090227.7, EP 07090007.1, EP 07090009.7, WO 2001/14569, WO 2002/79410, WO 2003/33540, WO 2004/078983, WO 2001/19975, WO 1995/26407, WO 1996/34968, WO 1998/20145, WO 1999/12950, WO 1999/66050, WO 1999/53072, US 6,734,341, WO 2000/11192, WO 1998/22604, WO 1998/32326, WO 2001/98509, WO 2001/98509, WO 2005/002359, US 5,824,790, US 6,013,861, WO 1994/004693, WO 1994/009144, WO 1994/11520, WO 1995/35026, WO 1997/20936.
2) transgenic plants which synthesize non starch carbohydrate polymers or which synthesize non starch carbohydrate polymers with altered properties in comparison to wild type plants without genetic modification. Examples are plants producing polyfructose, especially of the inulin and levan-type, as disclosed in EP 0663956, WO 1996/001904, WO 1996/021023, WO 1998/039460, and WO 1999/024593, plants producing alpha 1,4 glucans as disclosed in WO 1995/031553, US 2002/031826, US 6,284,479, US 5,712,107, WO 1997/047806, WO 1997/047807, WO 1997/047808 and WO 2000/014249, plants producing alpha-1,6 branched alpha-1,4-glucans, as disclosed in WO 2000/73422, plants producing alternan, as disclosed in WO 2000/047727, EP 06077301.7, US 5,908,975 and EP 0728213,
3) transgenic plants which produce hyaluronan, as for example disclosed in WO 2006/032538, WO 2007/039314, WO 2007/039315, WO 2007/039316, JP 2006/304779, and WO 2005/012529.

Plants or plant cultivars (that can be obtained by plant biotechnology methods such as genetic engineering) which may also be treated according to the invention are plants, such as cotton plants, with altered fiber characteristics. Such plants can be obtained by genetic transformation, or by selection of plants contain a mutation imparting such altered fiber characteristics and include:
a) Plants, such as cotton plants, containing an altered form of cellulose synthase genes as described in WO 1998/000549
b) Plants, such as cotton plants, containing an altered form of rsw2 or rsw3 homologous nucleic acids as described in WO2004/053219
c) Plants, such as cotton plants, with increased expression of sucrose phosphate synthase as described in WO 2001/017333
d) Plants, such as cotton plants, with increased expression of sucrose synthase as described in WO02/45485
e) Plants, such as cotton plants, wherein the timing of the plasmodesmatal gating at the basis of the fiber cell is altered, e.g. through downregulation of fiberselective β 1,3-glucanase as described in WO2005/017157
f) Plants, such as cotton plants, having fibers with altered reactivity, e.g. through the expression of N-acteylglucosaminetransferase gene including nodC and chitinsynthase genes as described in WO2006/136351

Plants or plant cultivars (that can be obtained by plant biotechnology methods such as genetic engineering) which may also be treated according to the invention are plants, such as oilseed rape or related Brassica plants, with altered oil profile characteristics. Such plants can be obtained by genetic transformation or by selection of plants contain a mutation imparting such altered oil characteristics and include:
a) Plants, such as oilseed rape plants, producing oil having a high oleic acid content as described e.g. in US 5,969,169, US 5,840,946 or US 6,323,392 or US 6,063,947
b) Plants such as oilseed rape plants, producing oil having a low linolenic acid content as described in US 6,270828, US 6,169,190 or US 5,965,755
c) Plant such as oilseed rape plants, producing oil having a low level of saturated fatty acids as described e.g. in US 5,434,283

Particularly useful transgenic plants which may be treated according to the invention are plants which comprise one or more genes which encode one or more toxins, such as the following which are sold under the trade names YIELD GARD® (for example maize, cotton, soya beans), KnockOut® (for example maize), BiteGard® (for example maize), Bt-Xtra® (for example maize), StarLink® (for example maize), Bollgard® (cotton), Nucotn® (cotton), Nucotn 33B®(cotton), NatureGard® (for example maize), Protecta® and NewLeaf® (potato). Examples of herbicide-tolerant plants which may be mentioned are maize varieties, cotton varieties and soya bean varieties which are sold under the trade names Roundup Ready® (tolerance to glyphosate, for example maize, cotton, soya bean), Liberty Link® (tolerance to phosphinotricin, for example oilseed rape), IMI® (tolerance to imidazolinones) and STS® (tolerance to sulphonylureas, for example maize). Herbicide-resistant plants (plants bred in a conventional manner for herbicide tolerance) which may be mentioned include the varieties sold under the name Clearfield® (for example maize).

Particularly useful transgenic plants which may be treated according to the invention are plants containing transformation events, or combination of transformation events, that are listed for example in the databases from various national or regional regulatory agencies (see for example http://gmoinfo.jrc.it/gmp_browse.aspx and http://www.agbios.com/dbase.php).

The composition according to the invention may also be used against fungal diseases liable to grow on or inside timber. The term "timber" means all types of species of wood, and all types of working of this wood intended for construction, for example solid wood, high-density wood, laminated wood, and plywood. The method for treating timber according to the invention mainly consists in contacting one or more compounds according to the invention or a composition according to the invention; this includes for example direct application, spraying, dipping, injection or any other suitable means.

Among the diseases of plants or crops that can be controlled by the method according to the invention, mention can be made of :
- Powdery mildew diseases such as :
   Blumeria diseases, caused for example by *Blumeria graminis* ;
   Podosphaera diseases, caused for example by *Podosphaera leucotricha* ;
   Sphaerotheca diseases, caused for example by *Sphaerotheca fuliginea* ;
   Uncinula diseases, caused for example by *Uncinula necator* ;
- Rust diseases such as :
   Gymnosporangium diseases, caused for example by *Gymnosporangium sabinae* ;
   Hemileia diseases, caused for example by *Hemileia vastatrix* ;
   Phakopsora diseases, caused for example by *Phakopsora pachyrhizi* or *Phakopsora meibomiae* ;
   Puccinia diseases, caused for example by *Puccinia recondita* ;
   Uromyces diseases, caused for example by *Uromyces appendiculatus* ;
- Oomycete diseases such as :
   Albugo diseases caused for example by *Albugo candida*;
   Bremia diseases, caused for example by *Bremia lactucae* ;
   Peronospora diseases, caused for example by *Peronospora pisi* or *P. brassicae* ;
   Phytophthora diseases, caused for example by *Phytophthora infestans* ;
   Plasmopara diseases, caused for example by Pla*smopara viticola* ;
   Pseudoperonospora diseases, caused for example by *Pseudoperonospora humuli* or
   *Pseudoperonospora cubensis* ;
   Pythium diseases, caused for example by *Pythium ultimum* ;
- Leafspot, leaf blotch and leaf blight diseases such as :
   Alternaria diseases, caused for example by *Alternaria solani* ;
   Cercospora diseases, caused for example by *Cercospora beticola* ;
   Cladiosporum diseases, caused for example by *Cladiosporium cucumerinum* ;
   Cochliobolus diseases, caused for example by *Cochliobolus sativus* ;
   Colletotrichum diseases, caused for example by *Colletotrichum lindemuthanium* ;
   Cycloconium diseases, caused for example by *Cycloconium oleaginum* ;
   Diaporthe diseases, caused for example by *Diaporthe citri* ;
   Drechslera, Syn: Helminthosporium) or *Cochliobolus miyabeanus*;
   Elsinoe diseases, caused for example by *Elsinoe fawcettii* ;
   Gloeosporium diseases, caused for example by *Gloeosporium laeticolor* ;
   Glomerella diseases, caused for example by *Glomerella cingulata* ;
   Guignardia diseases, caused for example by *Guignardia bidwelli* ;
   Leptosphaeria diseases, caused for example by *Leptosphaeria maculans* ; *Leptosphaeria nodorum* ;
   Magnaporthe diseases, caused for example by *Magnaporthe grisea* ;
   Mycosphaerella diseases, caused for example by *Mycosphaerella graminicola* ; *Mycosphaerella arachidicola ; Mycosphaerella fijiensis ;*
   Phaeosphaeria diseases, caused for example by *Phaeosphaeria nodorum* ;
   Pyrenophora diseases, caused for example by *Pyrenophora teres* ;
   Ramularia diseases, caused for example by *Ramularia collo-cygni* ;
   Rhynchosporium diseases, caused for example by *Rhynchosporium secalis* ;
   Septoria diseases, caused for example by *Septoria apii* or *Septoria lycopercisi* ;
   Typhula diseases, caused for example by *Typhula incarnata* ;
   Venturia diseases, caused for example by *Venturia inaequalis* ;
- Root and stem diseases such as :
   Corticium diseases, caused for example by *Corticium graminearum* ;
   Fusarium diseases, caused for example by *Fusarium oxysporum* ;
   Gaeumannomyces diseases, caused for example by *Gaeumannomyces graminis* ;
   Rhizoctonia diseases, caused for example by *Rhizoctonia solani* ;
   Sarocladium diseases caused for example by *Sarocladium oryzae;*
   Sclerotium diseases caused for example by *Sclerotium oryzae*;
   Tapesia diseases, caused for example by *Tapesia acuformis* ;
   Thielaviopsis diseases, caused for example by *Thielaviopsis basicola* ;
- Ear and panicle diseases including maize cob, such as :
   Alternaria diseases, caused for example by *Alternaria spp*. ;
   Aspergillus diseases, caused for example by *Aspergillus flavus* ;
   Cladosporium diseases, caused for example by *Cladosporium spp*. ;
   Claviceps diseases, caused for example by *Claviceps purpurea* ;
   Fusarium diseases, caused for example by *Fusarium culmorum* ;
   Gibberella diseases, caused for example by *Gibberella zeae* ;
   Monographella diseases, caused for example by *Monographella nivalis* ;
- Smut and bunt diseases such as :
   Sphacelotheca diseases, caused for example by *Sphacelotheca reiliana* ;
   Tilletia diseases, caused for example by *Tilletia caries* ;
   Urocystis diseases, caused for example by *Urocystis occulta* ;
   Ustilago diseases, caused for example by *Ustilago nuda* ;
- Fruit rot and mould diseases such as:
   Aspergillus diseases, caused for example by *Aspergillus flavus* ;
   Botrytis diseases, caused for example by *Botrytis cinerea* ;
   Penicillium diseases, caused for example by *Penicillium expansum* ;
   Rhizopus diseases caused by example by *Rhizopus stolonifer* Sclerotinia diseases, caused for example by *Sclerotinia sclerotiorum* ;
   Verticilium diseases, caused for example by *Verticilium alboatrum* ;
- Seed and soil borne decay, mould, wilt, rot and damping-off diseases such as :
   Alternaria diseases, caused for example by *Alternaria brassicicola*
   Aphanomyces diseases, caused for example by *Aphanomyces euteiches*
   Ascochyta diseases, caused for example by *Ascochyta lentis*
   Aspergillus diseases, caused for example by *Aspergillus flavus*
   Cladosporium diseases, caused for example by *Cladosporium herbarum*
   Cochliobolus diseases, caused for example by *Cochliobolus sativus*
   (Conidiaform: *Drechslera, Bipolaris Syn: Helminthosporium*);
   Colletotrichum diseases, caused for example by *Colletotrichum coccodes*;
   Fusarium diseases, caused for example by *Fusarium culmorum*;
   Gibberella diseases, caused for example by *Gibberella zeae*;
   Macrophomina diseases, caused for example by *Macrophomina* phaseolina
   Monographella diseases, caused for example by *Monographella nivalis*;
   Penicillium diseases, caused for example by *Penicillium expansum*
   Phoma diseases, caused for example by *Phoma lingam*
   Phomopsis diseases, caused for example by *Phomopsis sojae*;
   Phytophthora diseases, caused for example by Phytophthora cactorum;
   Pyrenophora diseases, caused for example by *Pyrenophora graminea*
   Pyricularia diseases, caused for example by *Pyricularia oryzae*;
   Pythium diseases, caused for example by *Pythium ultimum*;
   Rhizoctonia diseases, caused for example by *Rhizoctonia solani*;
   Rhizopus diseases, caused for example by *Rhizopus oryzae* Sclerotium diseases, caused for example by *Sclerotium rolfsii*;
   Septoria diseases, caused for example by *Septoria nodorum*;
   Typhula diseases, caused for example by *Typhula incarnata*;
   Verticillium diseases, caused for example by *Verticillium dahliae* ;
- Canker, broom and dieback diseases such as :
   Nectria diseases, caused for example by *Nectria galligena* ;
- Blight diseases such as :
   Monilinia diseases, caused for example by *Monilinia laxa* ;
- Leaf blister or leaf curl diseases such as :
   Exobasidium diseases caused for nexample by *Exobasidium vexans;*
   Taphrina diseases, caused for example by *Taphrina deformans* ;
- Decline diseases of wooden plants such as :
   Esca diseases, caused for example by *Phaemoniella clamydospora, Phaeomoniella clamydospora,*
   *Phaeoacremonium aleophilum and Fomitiporia mediterranea ;*
   Eutypa dyeback, caused for example by *Eutypa lata* ;
   Dutch elm disease, caused for example by *Ceratocystsc ulmi* ;
   Ganoderma diseases caused by example by Ganoderma boninense;
- Diseases of flowers and Seeds such as :
   Botrytis diseases, caused for example by *Botrytis cinerea* ;
- Diseases of tubers such as :
   Rhizoctonia diseases, caused for example by *Rhizoctonia solani*
   Helminthosporium diseases, caused for example by *Helminthosporium solani.*
- Diseases of Tubers such as
   Rhizoctonia diseases caused for example by *Rhizoctonia solani* ;
   Helminthosporium diseases caused for example by *Helminthosporium solani* ;
- Club root diseases such as
   Plasmodiophora diseases, caused for example by *Plamodiophora brassicae* ;
- Diseases caused by Bacterial Organisms such as
   Xanthomanas species for example *Xanthomonas campestris pv. oryzae*;
   Pseudomonas species for example *Pseudomonas syringae pv. lachrymans*;
   Erwinia species for example *Erwinia amylovora.*

The fungicide composition according to the invention can also be used against fungal diseases liable to grow on or inside timber. The term "timber" means all types of species of wood and all types of working of this wood intended for construction, for example solid wood, high-density wood, laminated wood and plywood. The method for treating timber according to the invention mainly consists in contacting one or more compounds according to the invention, or a composition according to the invention ; this includes for example direct application, spraying, dipping, injection or any other suitable means.

The dose of active compound usually applied in the method of treatment according to the invention is generally and advantageously from 10 to 800 g/ha, preferably from 50 to 300 g/ha for applications in foliar treatment. The dose of active substance applied is generally and advantageously from 2 to 200 g per 100 kg of seed, preferably from 3 to 150 g per 100 kg of seed in the case of seed treatment.

It is clearly understood that the doses indicated herein are given as illustrative examples of the method according to the invention. A person skilled in the art will know how to adapt the application doses, notably according to the nature of the plant or crop to be treated.

The fungicide composition according to the invention can also be used in the treatment of genetically modified organisms with the compounds according to the invention or the agrochemical compositions according to the invention. Genetically modified plants are plants into genome of that a heterologous gene encoding a protein of interest has been stably integrated. The expression "heterologous gene encoding a protein of interest" essentially means genes that give the transformed plant new agronomic properties, or genes for improving the agronomic quality of the modified plant.

The compounds or mixtures according to the invention can also be used for the preparation of composition useful to curatively or preventively treat human or animal fungal diseases such as, for example, mycoses, dermatoses, trichophyton diseases and candidiases or diseases caused by *Aspergillus spp.,* for example *Aspergillus fumigatus.*

The present invention also relates to a process for the preparation of compounds of formula (I).

The process according to the invention can be carried according to the following schemes and reaction conditions. Specific steps and particular conditions can be adapted by the skilled person in order to prepare the compounds of formula (I) according to the invention.

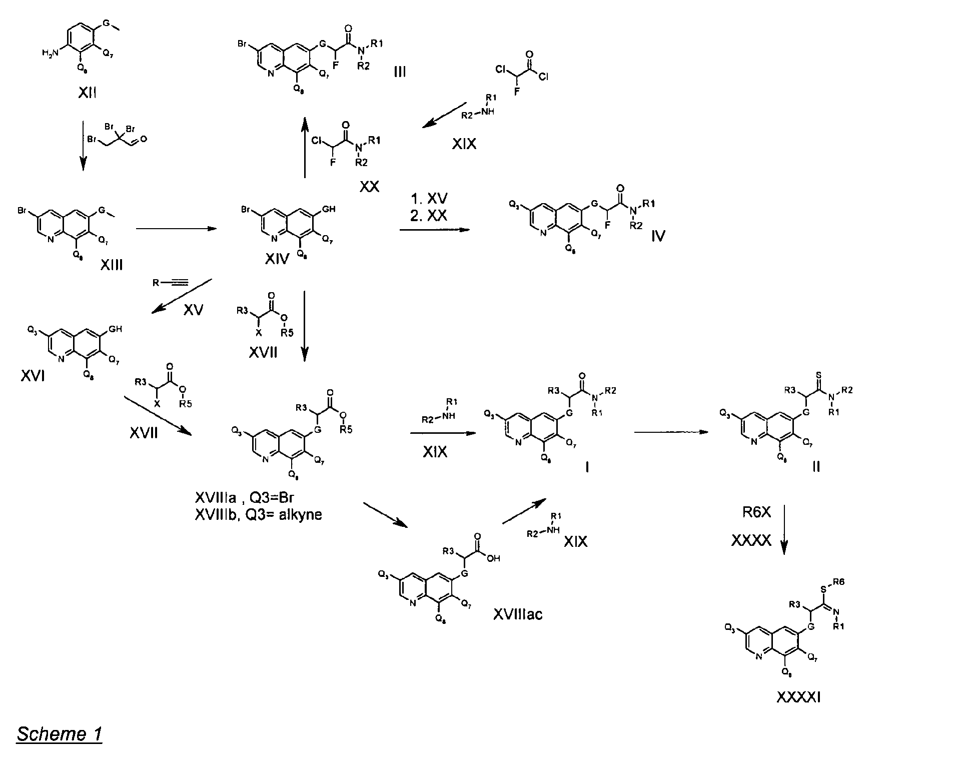

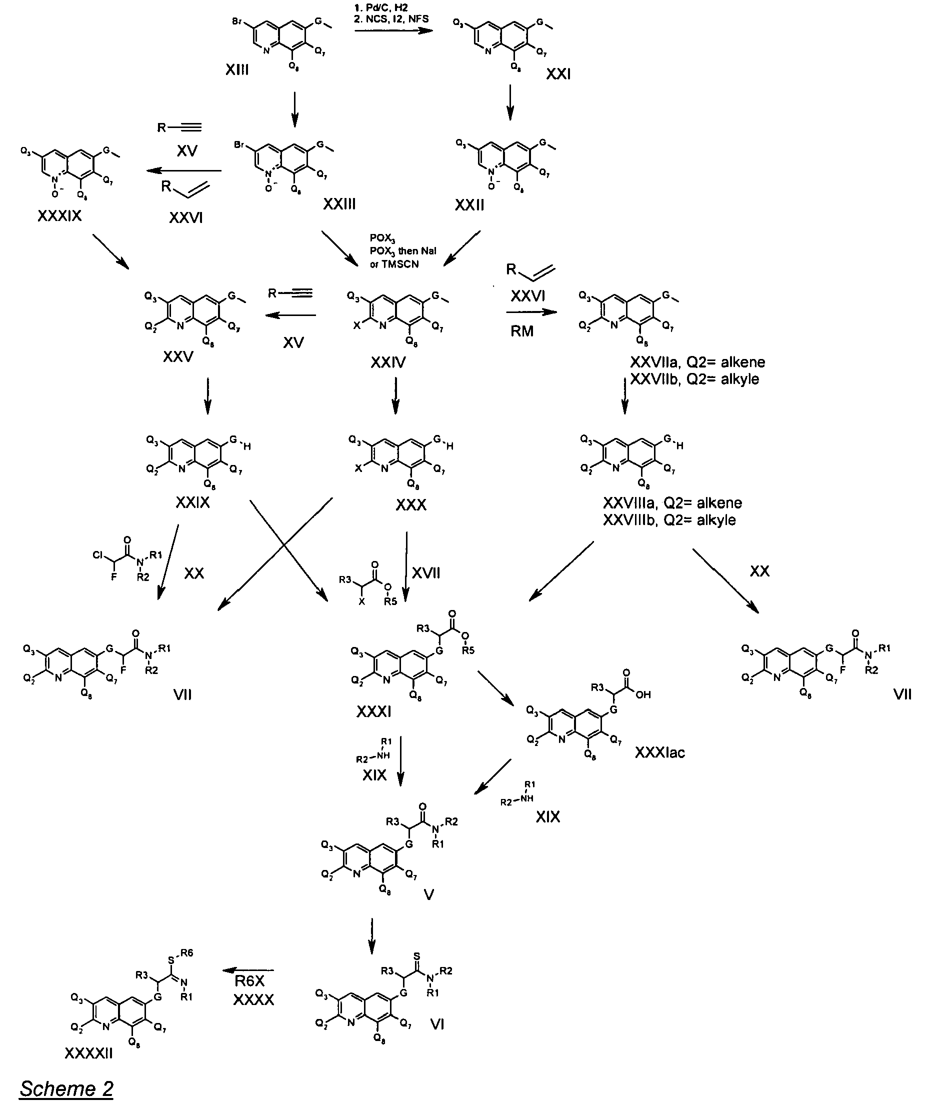

### Step XII → XIII

The quinoline derivatives are prepared from aniline derivatives and tribromopropanal as described in literature (WO2008028624, US4489098).

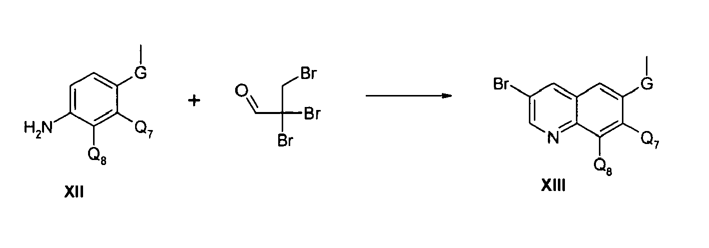

### Step XIII → XIV:

The protecting group of the function G (OH and SH) is cleaved under conditions known in the literature (Protective Groups in Organic Synthesis, T.W. Greene and P.G.M. Wuts, Wiley-Interscience, 1999; Garst et al J. Org. Chem. 2000, 65, 7098-7104; Cabiddu et al Synthesis 1982, 7, 583-584; Lavanish et al Tetrahedron Letters 1973, 39, 3847-3848).

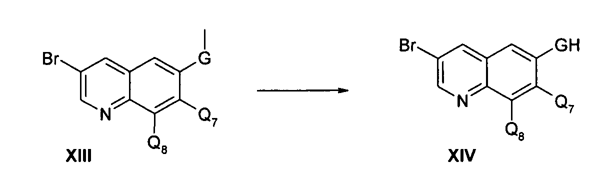

### Step XIV + XV→ XVI

The compounds XVI are prepared in analogy to procedures known and described in the literature, from the halogenoquinolines and alkynes in presence of catalysts (Chinchila et al. Chem Rev 2007, 107, 874-922). Furthermore, if R is a trimethylsilyl group, it is cleaved by methanolysis in presence of an inorganic base like potassium carbonate, caesium carbonate, lithium carbonate, calcium carbonate, or with trifluoroacetic acid.

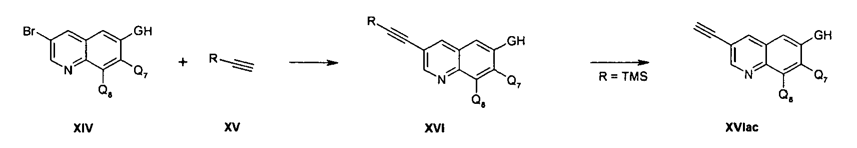

### Step XVI + XVII → XVIII, step XIV + XVII → XVIII, Step XVI + XX → IV

The ether is prepared from an alkyl halide and the hydroxyquinolines in the presence of a base in an inert solvent.

The inert solvents for the ether formation are the usual organic solvents: acetonitrile, dimethylformamide, dimethylsulfoxide, dimethylacetamide, N-methyl-2-pyrrolidone, tetrahydrofurane, diethylether, dioxane, toluene, hexane, cyclohexane. Dimethylformamide is preferred.

As bases are the usual inorganic bases suitable for the ether formation. These preferably include alkali hydroxide like sodium hydroxide, potassium hydroxide, lithium hydroxide or barium hydroxide, potassium tert-butyloxide or alkali carbonate like sodium carbonate, potassium carbonate, caesium carbonate or calcium carbonate. Potassium or caesium carbonate is preferred.

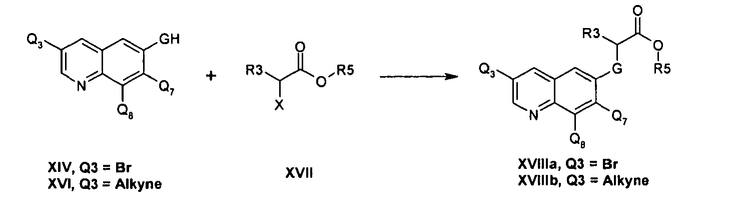

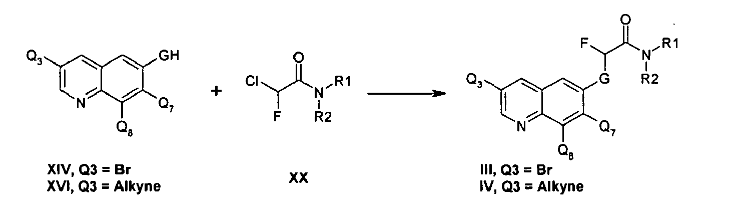

### Step XVIII → I

The ester is hydrolysed under usual methods, where the ester is treated with bases in inert solvents, the generated salts are then treated with acids to deliver the free carboxylic acids.

Water or the usual organic solvents for ester cleavage are suitable as inert solvents. These inert solvents are alcohols like methanol, ethanoln, propanol, isopropanol, n-butanol or tert-butanol ether like diethylether, tetrahydrofurane, dioxane or glycoldimethylether or other solvent like acetone, acetonitrile, dimethylformamide, dimethylsulfoxide. It is also possible to use a mixture of these solvents. In the case of a basic hydrolysis of esters, mixtures of water with dioxane, tetrahydrofurane, methanol and/or ethanol are preferred.

As bases are the usual inorganic bases suitable for the ester hydrolysis. These preferably include alkali hydroxide like sodium hydroxide, potassium hydroxide, lithium hydroxide or barium hydroxide or alkali carbonate like sodium carbonate, potassium carbonate, calcium carbonate or caesium carbonate. Sodium hydroxide or lithium hydroxide is preferred.

The ester hydrolysis is carried out at a temperature from 0°C to +100°C, preferentially from 0°C to +40°C. The reaction can be carried out at normal, high or low pressure (for example from 0.5 to 5 Bar). In general the reaction is carried out at normal pressure.

The carboxylic acid is then coupled with an amine. The coupling agent for the amide formation can be for example carbodiimide like N,N'-diethyl, N,N'-dipropyl, N,N'-diisopropyl, N,N'-dicyclohexylcarbodiimide (DCC), N-[3-(dimethylamino)propyl]-N'-ethylcarbodiimide hydrochloride (EDC), or N,N'-carbonyldiimidazol, or 1,2-oxazolium compounds like 2-ethyl-5-phenyl-1,2-oxazolium-3-sulfate or 2-tert.-butyl-5-methyl-isoxazolium-perchlorate or acylamino compounds like 2-ethoxy-1-ethoxycarbonyl-1,2-dihydrochinolin, or isobutylchloroformiate, 2,4,6-tripropyl-1,3,5,2,4,6-trioxatriphosphinane 2,4,6-trioxide, diethyl phosphorocyanidoate, bis-(2-oxo-3-oxazolidinyl)-phosphoryl chloride, (1H-benzotriazol-1-yloxy)[tris(dimethylamino)]phosphonium hexafluorophosphate, (1H-benzotriazol-1-yloxy)(tripyrrolidin-1-yl)phosphonium hexafluorophosphate (PyBOP), N-[(1H-benzotriazol-1-yloxy)(dimethylamino)methylidene]-N-methylmethanaminium tetrafluoroborate (TBTU), N-[(1H-benzotriazol-1-yloxy)(dimethylamino)methylidene]-N-methylmethanaminium hexafluorophosphate (HBTU), N-{(dimethylamino)[(2-oxopyridin-1(2H)-yl)oxy]methylidene}-N-methylmethanaminium tetrafluoroborate (TPTU), N-[(dimethylamino)(3H-[1,2,3]triazolo[4,5-b]pyridin-3-yloxy)methylidene]-N-methylmethanaminium hexafluorophosphate (HATU) or 1-[bis(dimethylamino)methylidene]-5-chloro-1H-benzotriazol-1-ium 3-oxide tetrafluoroborate (TCTU), if needed in combination with other derivative like 1H-benzotriazol-1-ol (HOBt) or N-hydroxysuccinimide (HOSu), as well as bases alkali carbonates, for example sodium or potassium carbonate, or sodium or potassium hydrogenocarbonate, or organic bases like trialkylamine, N-methylmorpholine, N-methylpiperidine or N,N-diisopropylethylamine.

This step is carried out in general in a temperature from -20°C to +60°C, preferentially 0°C to +40°C. The reaction can be carried out at normal, high or low pressure (for example from 0.5 to 5 Bar). In general the reaction is carried out at normal pressure.

Amides can be directly prepared in analogy to known procedures in the literature from esters and amines catalysed by a Lewis acids (MgBr2, MgCl2, Z. Guo et all Tetrahedron Letters, 2001, 42, 1843; AIMe3, Woodward et al Tetrahedron Letters 2006, 47, 5767, Tetrahedron Letters 2008 49, 5687) under reflux or micro wave irradiation (S. Woodward et al Tetrahedron Letters 2008 49, 5687).

Amides are directly prepared by treating esters with amines in presence of trialkylaluminium in an inert solvent.

The trialkylaluminium can be for example triethylaluminium, triisobutylaluminium, trimethylaluminium, tripropylaluminium, tri-tert-butylaluminum, tributylaluminum. Trimethylaluminium is preferred.

The organic solvents for direct amidation are ether like diethylether, tetrahydrofurane, dioxane or glycoldimethylether or other solvent like toluene. Tetrahydrofurane and toluene are preferred solvents. This step is carried out in general in a temperature range from 0°C to boiling point, preferentially boiling point. The reaction can be carried out at normal, high or low pressure (for example from 0.5 to 5 Bar). In general the reaction is carried out at normal pressure.

The amidation can also be carried out under micro wave irradiation.

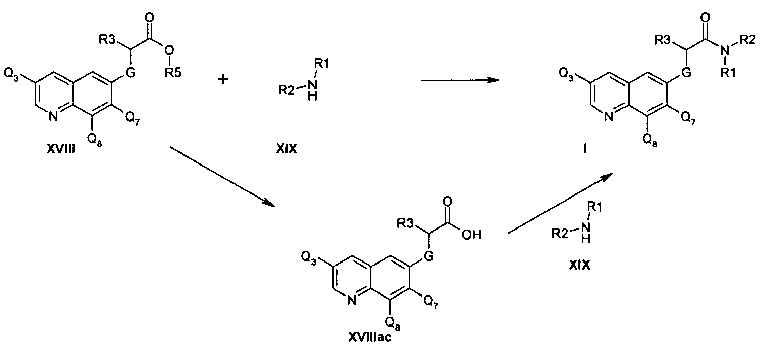

### Step I → II:

The thioamide II is prepared by treating the corresponding amide with thionation reagent in acetonitrile, tetrahydrofurane, diglyme, toluene.

The thionation reagent can be for example phosphorus pentasulfide, 2,4-bis(4-methoxyphenyl)-1,3,2,4-dithiadiphosphetane 2,4-disulfite (Lawesson's reagent).

### Step II + XXXX → XXXXI

The compounds XXXXI are prepared out in analogy to procedures described in the literature by treating the thioamides II with a base followed by an alkyl halide (Bartsch et al Monatshefte fuer Chemie 1988, 119, 1439-1444; Bernath et al Tetrahedron 1987, 43, 4359-4366; O'Gorman et al Tetrahedron Letters 2008, 49, 6316-6319; Takahata et al Heterocycles 1985, 23, 2219-2215; Takahata et al J. Org. Chem 1990, 55, 3792-3797).

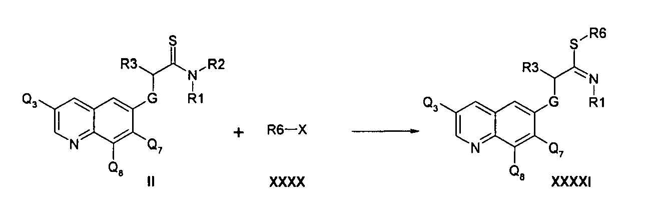

### Preparation of XX

The chlorofluoroacetylamide XX is prepared by treating the commercial chlorofluoroacetyl chloride with an amine in the presence of a base in an inert solvent.

As bases are the usual organic bases. These preferably include trialkylamine like triethylamine, diisopropylamine, or pyridine, 2,6-lutidine .

Inert solvents are ether like diethylether, tetrahydrofurane, dioxane or glycoldimethylether or other solvent like acetonitrile, dimethylformamide, dimethylsulfoxide, chloroform, dichloromethane.

The reaction is carried out at a temperature preferentially from 0°C to +40°C.

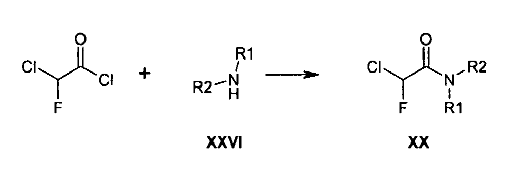

### Preparation of XVII

XVII represents: 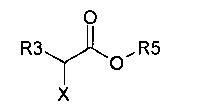 Methyl bromo(cyano)acetate is prepared according the procedures known and described in the literature (Zhang et al Chinese Chemical Letters 2005, 16, 1590-1592; Heravi et al South African Journal of Chemistry 2006, 59, 125-128; Lakouraj et al Journal of Chemical Research 2005, 8, 481-483; Khachatryan et al Armyanskii Khimicheskii Zhurnal 1986, 39(5), 290-294)

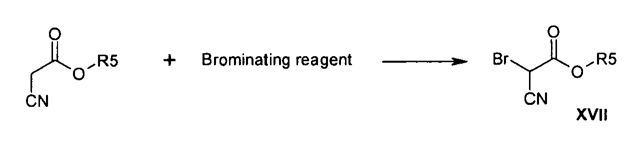

Methyl chloro(azolo)acetate is prepared by treating methyl bromochloroacetate with azole in analogy to procedures known in the literature (Ichijima et al DE3518797)

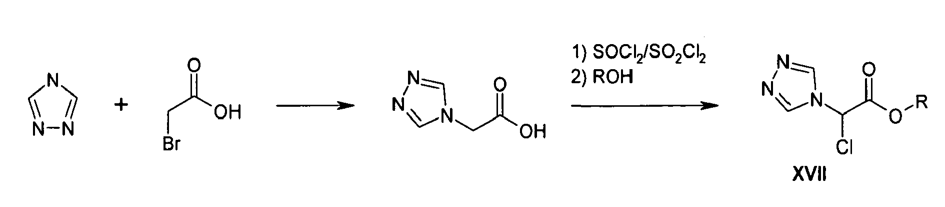

### Step XIII → XXIII

The step XIII → XXIII is carried out with 3-chloroperoxybenzoic acid, Hydrogen peroxide, or trioxirane, in a solvent like dichloromethane, chloroform or acetic acid, water. 3-Chloroperoxybenzoic acid is preferred.

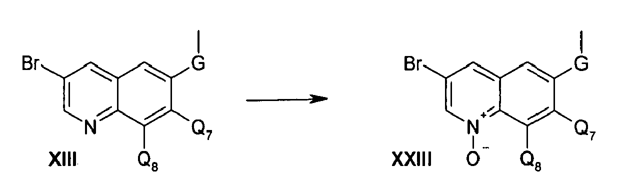

### Step XXIII → XXIV

The halogenation in the step XXIII → XXIV is carried out with phosphoric trihalide like phosphoric trichloride, phosphoric tribromide, phosphoric trichloride then Sodium iodide, or trimethylsilanecarbonitrile or with sulfuryl dichloride, thionyl dichloride with or without solvent like toluene, chloroform, dichloromethane.

The reaction is carried out at a temperature preferentially from 0°C to 120°C.

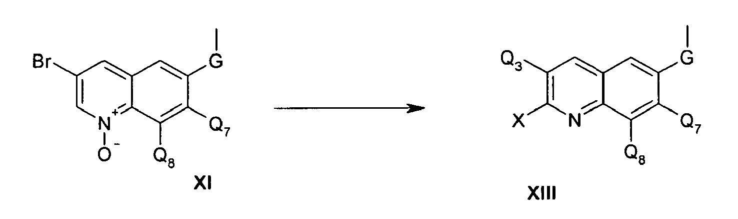

### Step XXIII → XXXIX

The compounds XXOXIX are prepared in analogy to procedures described in the literature by treating the 2-halogenated quinolines with organozinc reagents (Takahiro et al Heterocycles 2009, 77, 233-239; Hoekstra et al. J. Med. Chem. 2005, 48, 2243-2247) in presence of palladium catalysts, or with Grignard reagent in the presence of copper salts (Bell et al. J. Org. Chem. 1987, 52, 3847-50) or from the halogenoquinolines and alkynes in presence of catalysts (Chinchila et al. Chem Rev 2007, 107, 874-922).

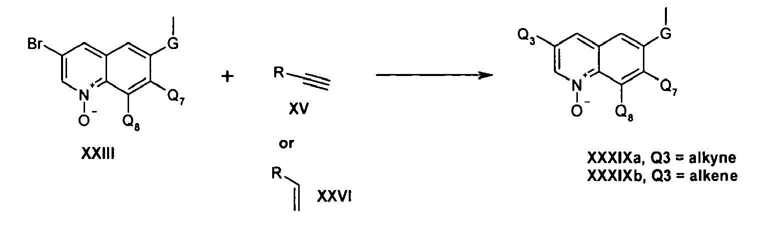

### Step XIII → XXI

Other halogenated quinolines are prepared by treating the halogenoquinolines XIII with hydrogene in the presence of a catalyst followed by treating with N-chloro-succinimide, N-iodo-succinimide, N-fluoro-succinimide, 12. The catalyst can be palladium on charcoal, platinum oxide, Raney nickel. Palladium on charcoal is the preferred catalyst.

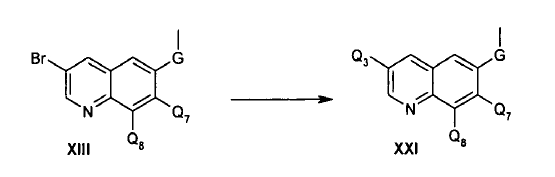

### Step XXI → XXII

The step XXI → XXII is carried out with 3-chloroperoxybenzoic acid, Hydrogen peroxide, or trioxirane, a solvent like dichloromethane, chloroform or acetic acid, water. 3-Chloroperoxybenzoic acid is preferred.

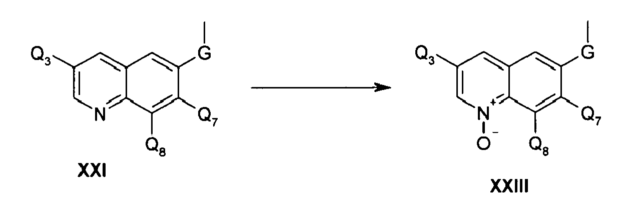

### Step XXII → XXIV

The step XXII → XXIV is carried out with phosphoric trihalide like phosphoric trichloride, phosphoric tribromide, phosphoric trichloride then Sodium iodide, or trimethylsilanecarbonitrile or with sulfuryl dichloride, thionyl dichloride with or without solvent like toluene, chloroform, dichloromethane.

The reaction is carried out at a temperature preferentially from 0°C to 120°C.

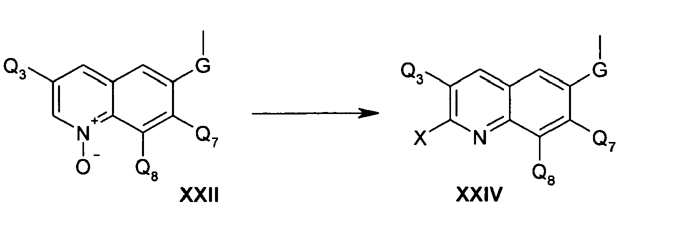

### Step XXIV + XV → XXV

The compounds XXV are prepared in analogy to procedures known in the literature, from the halogenoquinolines and alkynes catalysed by palladium (Chinchila et al Chem Rev 2007, 107, 874-922). Furthermore, if R is a trimethylsilyl group, it is cleaved by methanolysis in presence of an inorganic base like potassium carbonate, caesium carbonate, lithium carbonate, calcium carbonate, or with trifluoroacetic acid.

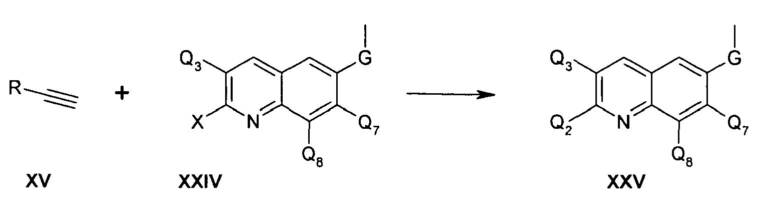

### Step XXIV → XXVIIa

The compounds XXVIIa are prepared in analogy to procedures described in the literature by treating the 2-halogenated quinolines with organozinc reagents (Takahiro et al Heterocycles 2009, 77, 233-239), alkenylchlorosilanes (Hagiwara et al Tetrahedron Letters 1997, 38, 439-442), alkenylsilanolates (Denmark et al J. Am. Chem. Soc. 2006, 128, 15958-15959) in presence of palladium catalysts.

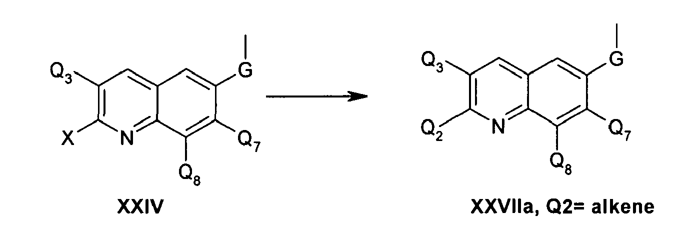

### Step XXIV → XXVIIb

The compounds XXVIIb are prepared in analogy to procedures described in the literature by treating the 2-halogenated quinolines with organozinc reagents (Takahiro et al Heterocycles 2009, 77, 233-239; Hoekstra et al. J. Med. Chem. 2005, 48, 2243-2247) in presence of palladium catalysts, or with Grignard reagent in the presence of copper salts (Bell et al. J. Org. Chem. 1987, 52, 3847-50).

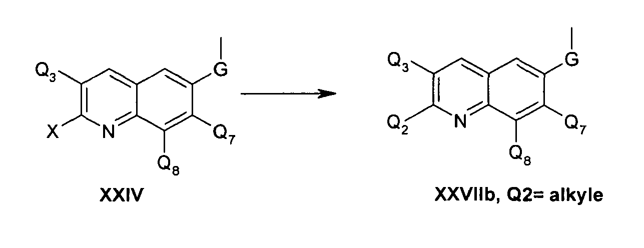

### Step XXV → XXIX, XXIV → XXX, XXVII → XXVIII

The protecting group of the function G (OH and SH) is cleaved under conditions known in the literature (Protective Groups in Organic Synthesis, T.W. Greene and P.G.M. Wuts, Wiley-Interscience, 1999; Garst et al J. Org. Chem. 2000, 65, 7098-7104; Cabiddu et al Synthesis 1982, 7, 583-584; Lavanish et al Tetrahedron Letters 1973, 39, 3847-3848).

### Step XXIX + XVII → XXI, XXX + XVII → XXXI, XXVIII + XVII → XXXI

The compound XXXI is prepared from an alkyl halide and the hydroxyquinolines in the presence of a base in an inert solvent.

The inert solvents for the ether formation are the usual organic solvents: acetonitrile, dimethylformamide, dimethylsulfoxide, dimethylacetamide, N-methyl-2-pyrrolidone, tetrahydrofurane, diethylether, dioxane, toluene, hexane, cyclohexane. Dimethylformamide is preferred.

As bases are the usual inorganic bases suitable for the ether formation. These preferably include alkali hydroxide like sodium hydroxide, potassium hydroxide, lithium hydroxide or barium hydroxide, potassium *tert*-butyloxide or alkali carbonate like sodium carbonate, potassium carbonate, caesium carbonate or calcium carbonate. Potassium or caesium carbonate is preferred.

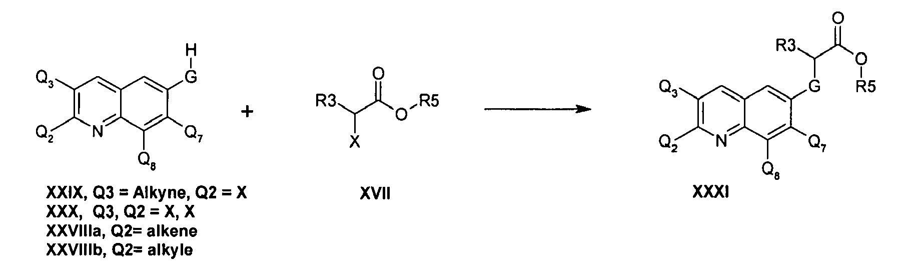

### Step XXXI → V

The ester is hydrolysed under usual methods, where the ester is treated with bases in inert solvents, the generated salts are then treated with acids to deliver the free carboxylic acids.

Water or the usual organic solvents for ester cleavage are suitable as inert solvents. These inert solvents are alcohols like methanol, ethanoln, propanol, isopropanol, n-butanol or tert-butanol ether like diethylether, tetrahydrofurane, dioxane or glycoldimethylether or other solvent like acetone, acetonitrile, dimethylformamide, dimethylsulfoxide. It is also possible to use a mixture of these solvents. In the case of a basic hydrolysis of esters, mixtures of water with dioxane, tetrahydrofurane, methanol and/or ethanol are preferred.

As bases are the usual inorganic bases suitable for the ester hydrolysis. These preferably include alkali hydroxide like sodium hydroxide, potassium hydroxide, lithium hydroxide or barium hydroxide or alkali carbonate like sodium carbonate, potassium carbonate, calcium carbonate or caesium carbonate. Sodium hydroxide or lithium hydroxide is preferred.

The ester hydrolysis is carried out at a temperature from 0°C to +100°C, preferentially from 0°C to +40°C. The reaction can be carried out at normal, high or low pressure (for example from 0.5 to 5 Bar). In general the reaction is carried out at normal pressure.

The carboxylic acid is then coupled with an amine. The coupling agent for the amide formation can be for example carbodiimide like N,N'-diethyl, N,N'-dipropyl, N,N'-diisopropyl, N,N'-dicyclohexylcarbodiimide (DCC), N-[3-(dimethylamino)propyl]-N'-ethylcarbodiimide hydrochloride (EDC), or N,N'-carbonyldiimidazol, or 1,2-oxazolium compounds like 2-ethyl-5-phenyl-1,2-oxazolium-3-sulfate or 2-tert.-butyl-5-methyl-isoxazolium-perchlorate or acylamino compounds like 2-ethoxy-1-ethoxycarbonyl-1,2-dihydrochinolin, or isobutylchloroformiate, 2,4,6-tripropyl-1,3,5,2,4,6-trioxatriphosphinane 2,4,6-trioxide, diethyl phosphorocyanidoate, bis-(2-oxo-3-oxazolidinyl)-phosphoryl chloride, (1H-benzotriazol-1-yloxy)[tris(dimethylamino)]phosphonium hexafluorophosphate, (1H-benzotriazol-1-yloxy)(tripyrrolidin-1-yl)phosphonium hexafluorophosphate (PyBOP), N-[(1H-benzotriazol-1-yloxy)(dimethylamino)methylidene]-N-methylmethanaminium tetrafluoroborate (TBTU), N-[(1H-benzotriazol-1-yloxy)(dimethylamino)methylidene]-N-methylmethanaminium hexafluorophosphate (HBTU), N-{(dimethylamino)[(2-oxopyridin-1(2H)-yl)oxy]methylidene}-N-methylmethanaminium tetrafluoroborate (TPTU), N-[(dimethylamino)(3H-[1,2,3]triazolo[4,5-b]pyridin-3-yloxy)methylidene]-N-methylmethanaminium hexafluorophosphate (HATU) or 1-[bis(dimethylamino)methylidene]-5-chloro-1H-benzotriazol-1-ium 3-oxide tetrafluoroborate (TCTU), if needed in combination with other derivative like 1H-benzotriazol-1-ol (HOBt) or N-hydroxysuccinimide (HOSu), as well as bases alkali carbonates, for example sodium or potassium carbonate, or sodium or potassium hydrogenocarbonate, or organic bases like trialkylamine, N-methylmorpholine, N-methylpiperidine or N,N-diisopropylethylamine.

This step is carried out in general in a temperature from -20°C to +60°C, preferentially 0°C to +40°C. The reaction can be carried out at normal, high or low pressure (for example from 0.5 to 5 Bar). In general the reaction is carried out at normal pressure.

Amides can be directly prepared in analogy to known procedures in the literature from esters and amines catalysed by a Lewis acids (MgBr2, MgCl2, Z. Guo et all Tetrahedron Letters, 2001, 42, 1843; AIMe3, Woodward et al Tetrahedron Letters 2006, 47, 5767, Tetrahedron Letters 2008 49, 5687) under reflux or micro wave irradiation (S. Woodward et al Tetrahedron Letters 2008 49, 5687).

Amides are directly prepared by treating esters with amines in presence of trialkylaluminium in an inert solvent.

The trialkylaluminium can be for example triethylaluminium, triisobutylaluminium, trimethylaluminium, tripropylaluminium, tri-tert-butylaluminum, tributylaluminum. Trimethylaluminium is preferred.

The organic solvents for direct amidation are ether like diethylether, tetrahydrofurane, dioxane or glycoldimethylether or other solvent like toluene. Tetrahydrofurane and toluene are preferred solvents. This step is carried out in general in a temperature from 0°C to boiling point, preferentially boiling point. The reaction can be carried out at normal, high or low pressure (for example from 0.5 to 5 Bar). In general the reaction is carried out at normal pressure.

The amidation can also be carried out under micro wave irradiation.

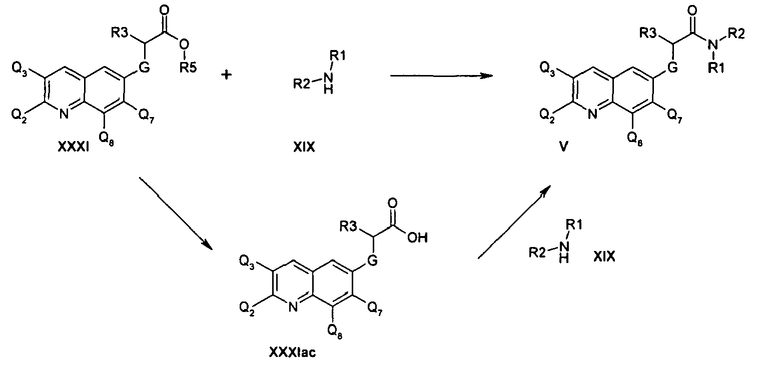

### Step V → VI

The thioamide VI is prepared by treating the corresponding amide with thionation reagent in acetonitrile, tetrahydrofurane, diglyme, toluene.

The thionation reagent can be for example phosphorus pentasulfide, 2,4-bis(4-methoxyphenyl)-1,3,2,4-dithiadiphosphetane 2,4-disulfite (Lawesson's reagent).

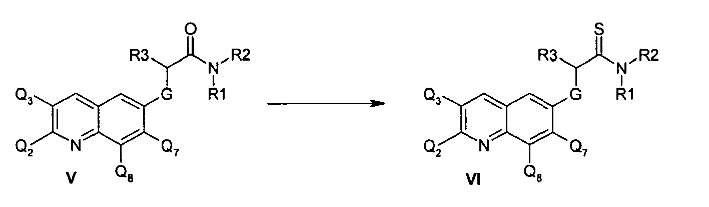

### Step VI + XXXX → XXXXII

The compounds XXXXII are prepared out in analogy to procedures described in the literature by treating the thioamides II with a base followed by an alkyl halide (Bartsch et al Monatshefte fuer Chemie 1988, 119, 1439-1444; Bernath et al Tetrahedron 1987, 43, 4359-4366; O'Gorman et al Tetrahedron Letters 2008, 49, 6316-6319; Takahata et al Heterocycles 1985, 23, 2219-2215; Takahata et al J. Org. Chem 1990, 55, 3792-3797).

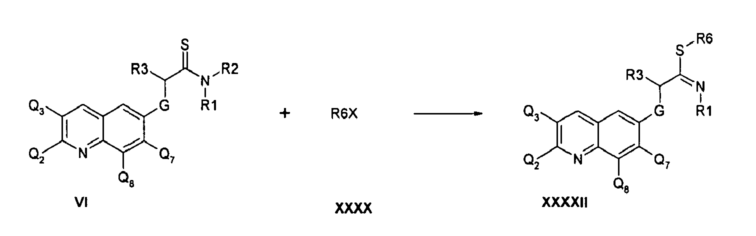

### Step XXIX + XX → VII, XXX + XX → VII, XXVIII → VII

The compound VII is prepared from an alkyl halide and the hydroxyquinolines in the presence of a base in an inert solvent.

The inert solvents for the ether formation are the usual organic solvents: acetonitrile, dimethylformamide, dimethylsulfoxide, dimethylacetamide, N-methyl-2-pyrrolidone, tetrahydrofurane, diethylether, dioxane, toluene, hexane, cyclohexane. Dimethylformamide is preferred.

Bases are the usual inorganic bases suitable for the ether formation. These preferably include alkali hydroxide like sodium hydroxide, potassium hydroxide, lithium hydroxide or barium hydroxide, potassium *tert*-butyloxide or alkali carbonate like sodium carbonate, potassium carbonate, caesium carbonate or calcium carbonate. Potassium or caesium carbonate is preferred.

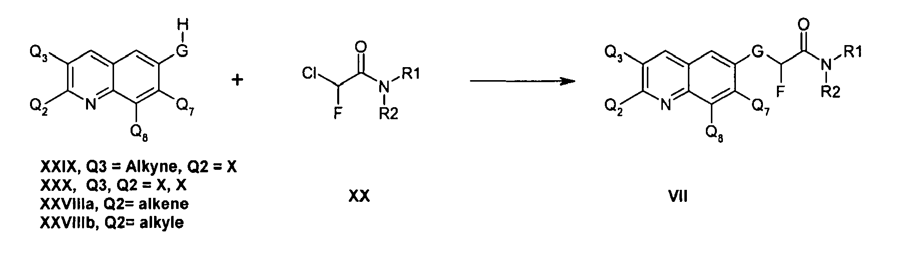

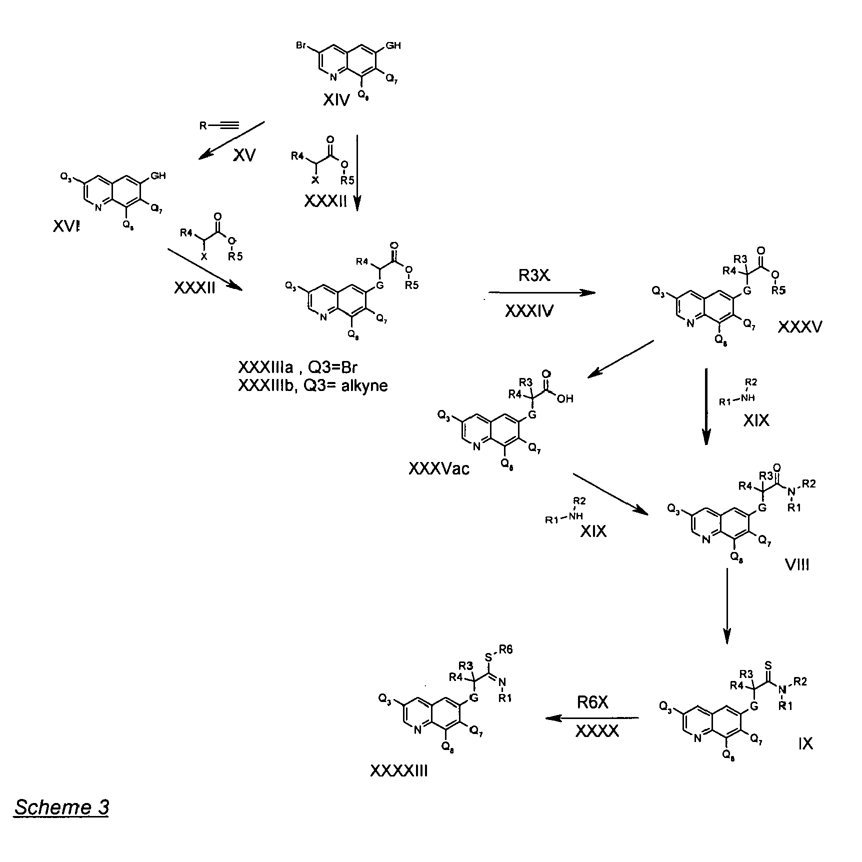

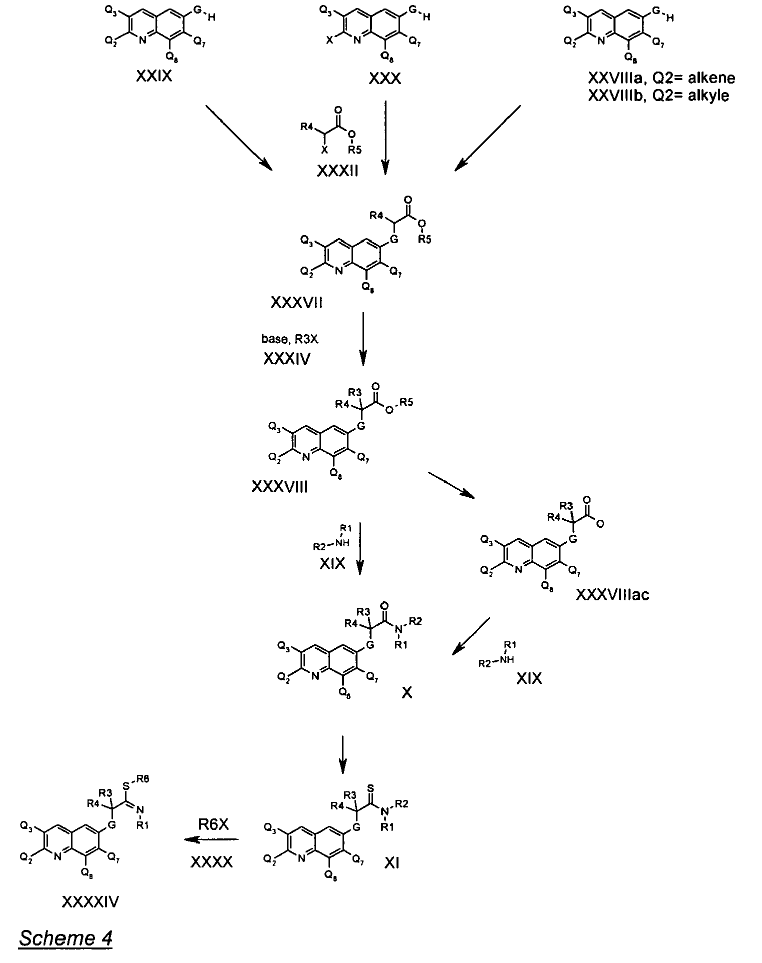

### Step XVI + XXXII → XXXIII, step XIV + XXXII → XXXIII

The ether is prepared from an alkyl halide and the hydroxyquinolines in the presence of a base in an inert solvent.

The inert solvents for the ether formation are the usual organic solvents: acetonitrile, dimethylformamide, dimethylsulfoxide, dimethylacetamide, N-methyl-2-pyrrolidone, tetrahydrofurane, diethylether, dioxane, toluene, hexane, cyclohexane. Dimethylformamide is preferred.

As bases are the usual inorganic bases suitable for the ether formation. These preferably include alkali hydroxide like sodium hydroxide, potassium hydroxide, lithium hydroxide or barium hydroxide, potassium tert-butyloxide or alkali carbonate like sodium carbonate, potassium carbonate, caesium carbonate or calcium carbonate. Potassium or caesium carbonate is preferred.

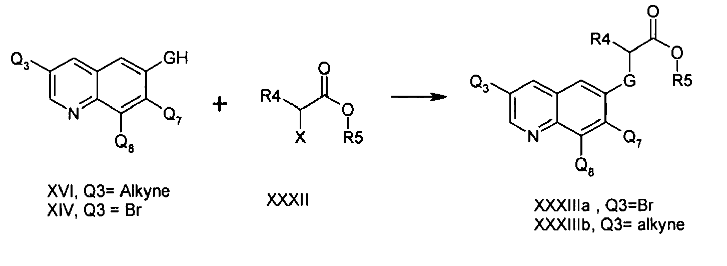

### Step XXXIII + XXXIV → XXXV

The step XXXIII + XXXIV → XXXV can be prepared in analogy to known procedures of the literature ( Ma et al. J. Org. Chem. 2003, 68, 8726-8729; Umemoto et al. Chem. Res. Cent., J. Am. Chem. Soc. 1993, 115, 2156-2164; Yang et al. Synlett 1997, 2, 1379-1380; Bretschneider,H. et al. Monatshefte fuer Chemie 1965, 96, 1661 - 1676; Bendich J. Am. Chem. Soc.1953, 75, 4075; Luthman et al. J. Org. Chem. 1987, 52, 3777-3784).

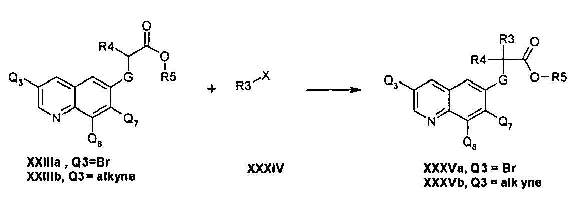

Inert solvents for the step XXXIII + XXXIV → XXXV are for example ether like diethyl ether, dioxane, tetrahydrofurane, glycoldimethylether or diethylenglycoldimethylether or other solvent toluene, hexane, cyclohexane. It is also possible to use a mixture of these solvents. Tetrahydrofurane is preferred.

As bases for the step XXXIII + XXXIV → XXXV are for example bases like n-butyllithium, sec-butyllithium, tert-butyllithium, lithium diisopropylamide, lithium hexamethyldisilazane, potassium hexamethyldisilazane, sodium hexamethyldisilazane.

### Step XXXV + XIX → VIII

The ester is hydrolysed under usual methods, where the ester is treated with bases in inert solvents, the generated salts are then treated with acids to deliver the free carboxylic acids.

Water or the usual organic solvents for ester cleavage are suitable as inert solvents. These inert solvents are alcohols like methanol, ethanoln, propanol, isopropanol, n-butanol or tert-butanol ether like diethylether, tetrahydrofurane, dioxane or glycoldimethylether or other solvent like acetone, acetonitrile, dimethylformamide, dimethylsulfoxide. It is also possible to use a mixture of these solvents. In the case of a basic hydrolysis of esters, mixtures of water with dioxane, tetrahydrofurane, methanol and/or ethanol are preferred.

As bases are the usual inorganic bases suitable for the ester hydrolysis. These preferably include alkali hydroxide like sodium hydroxide, potassium hydroxide, lithium hydroxide or barium hydroxide or alkali carbonate like sodium carbonate, potassium carbonate, calcium carbonate or caesium carbonate. Sodium hydroxide or lithium hydroxide is preferred.

The ester hydrolysis is carried out at a temperature from 0°C to +100°C, preferentially from 0°C to +40°C. The reaction can be carried out at normal, high or low pressure (for example from 0.5 to 5 Bar). In general the reaction is carried out at normal pressure.

The carboxylic acid is then coupled with an amine. The coupling agent for the amide formation can be for example carbodiimide like N,N'-diethyl, N,N'-dipropyl, N,N'-diisopropyl, N,N'-dicyclohexylcarbodiimide (DCC), N-[3-(dimethylamino)propyl]-N'-ethylcarbodiimide hydrochloride (EDC), or N,N'-carbonyldiimidazol, or 1,2-oxazolium compounds like 2-ethyl-5-phenyl-1,2-oxazolium-3-sulfate or 2-tert.-butyl-5-methyl-isoxazolium-perchlorate or acylamino compounds like 2-ethoxy-1-ethoxycarbonyl-1,2-dihydrochinolin, or isobutylchloroformiate, 2,4,6-tripropyl-1,3,5,2,4,6-trioxatriphosphinane 2,4,6-trioxide, diethyl phosphorocyanidoate, bis-(2-oxo-3-oxazolidinyl)-phosphoryl chloride, (1H-benzotriazol-1-yloxy)[tris(dimethylamino)]phosphonium hexafluorophosphate, (1H-benzotriazol-1-yloxy)(tripyrrolidin-1-yl)phosphonium hexafluorophosphate (PyBOP), N-[(1H-benzotriazol-1-yloxy)(dimethylamino)methylidene]-N-methylmethanaminium tetrafluoroborate (TBTU), N-[(1H-benzotriazol-1-yloxy)(dimethylamino)methylidene]-N-methylmethanaminium hexafluorophosphate (HBTU), N-{(dimethylamino)[(2-oxopyridin-1(2H)-yl)oxy]methylidene}-N-methylmethanaminium tetrafluoroborate (TPTU), N-[(dimethylamino)(3H-[1,2,3]triazolo[4,5-b]pyridin-3-yloxy)methylidene]-N-methylmethanaminium hexafluorophosphate (HATU) or 1-[bis(dimethylamino)methylidene]-5-chloro-1H-benzotriazol-1-ium 3-oxide tetrafluoroborate (TCTU), if needed in combination with other derivative like 1H-benzotriazol-1-ol (HOBt) or N-hydroxysuccinimide (HOSu), as well as bases alkali carbonates, for example sodium or potassium carbonate, or sodium or potassium hydrogenocarbonate, or organic bases like trialkylamine, N-methylmorpholine, N-methylpiperidine or N,N-diisopropylethylamine.

This step is carried out in general in a temperature from -20°C to +60°C, preferentially 0°C to +40°C. The reaction can be carried out at normal, high or low pressure (for example from 0.5 to 5 Bar). In general the reaction is carried out at normal pressure.

Amides can be directly prepared in analogy to known procedures in the literature from esters and amines catalysed by a Lewis acids (MgBr2, MgCl2, Z. Guo et all Tetrahedron Letters, 2001, 42, 1843; AIMe3, Woodward et al Tetrahedron Letters 2006, 47, 5767, Tetrahedron Letters 2008 49, 5687) under reflux or micro wave irradiation (S. Woodward et al Tetrahedron Letters 2008 49, 5687).

Amides are directly prepared by treating esters with amines in presence of trialkylaluminium in an inert solvent.

The trialkylaluminium can be for example triethylaluminium, triisobutylaluminium, trimethylaluminium, tripropylaluminium, tri-tert-butylaluminum, tributylaluminum. Trimethylaluminium is preferred.

The organic solvents for direct amidation are ether like diethylether, tetrahydrofurane, dioxane or glycoldimethylether or other solvent like toluene. Tetrahydrofurane and toluene are preferred solvents. This step is carried out in general in a temperature from 0°C to boiling point, preferentially boiling point. The reaction can be carried out at normal, high or low pressure (for example from 0.5 to 5 Bar). In general the reaction is carried out at normal pressure.

The amidation can also be carried out under micro wave irradiation.

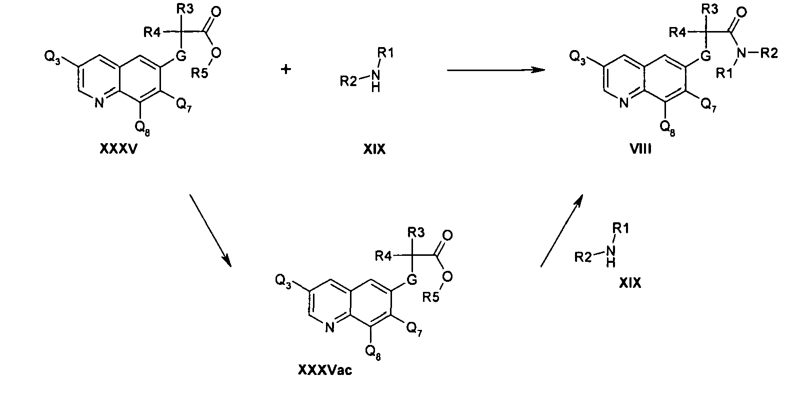

### Step VIII → IX

The thioamide IX is prepared by treating the corresponding amide with thionation reagent in acetonitrile, tetrahydrofurane, diglyme, toluene.

The thionation reagent can be for example phosphorus pentasulfide, 2,4-bis(4-methoxyphenyl)-1,3,2,4-dithiadiphosphetane 2,4-disulfite (Lawesson's reagent).

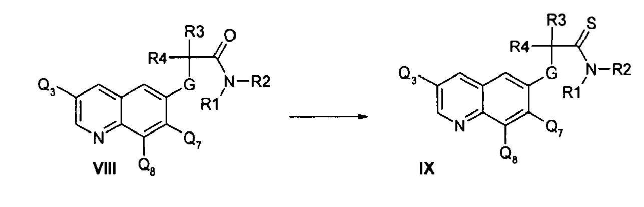

### Step IX + XXXX → XXXXIII

The compounds XXXXIII are prepared out in analogy to procedures described in the literature by treating the thioamides II with a base followed by an alkyl halide (Bartsch et al Monatshefte fuer Chemie 1988, 119, 1439-1444; Bernath et al Tetrahedron 1987, 43, 4359-4366; O'Gorman et al Tetrahedron Letters 2008, 49, 6316-6319; Takahata et al Heterocycles 1985, 23, 2219-2215; Takahata et al J. Org. Chem 1990, 55, 3792-3797).

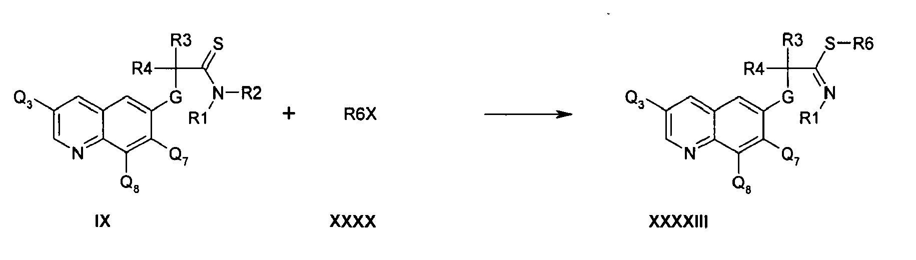

### Step XXIX + XXXVI → XXXVII, XXX + XXXVI → XXXVI, XXVIII + XXXVI → XXXVI

The ether is prepared from an alkyl halide and the hydroxyquinolines in the presence of a base in an inert solvent.

The inert solvents for the ether formation are the usual organic solvents: acetonitrile, dimethylformamide, dimethylsulfoxide, dimethylacetamide, N-methyl-2-pyrrolidone, tetrahydrofurane, diethylether, dioxane, toluene, hexane, cyclohexane. Dimethylformamide is preferred.

As bases are the usual inorganic bases suitable for the ether formation. These preferably include alkali hydroxide like sodium hydroxide, potassium hydroxide, lithium hydroxide or barium hydroxide, potassium tert-butyloxide or alkali carbonate like sodium carbonate, potassium carbonate, caesium carbonate or calcium carbonate. Potassium or caesium carbonate is preferred.

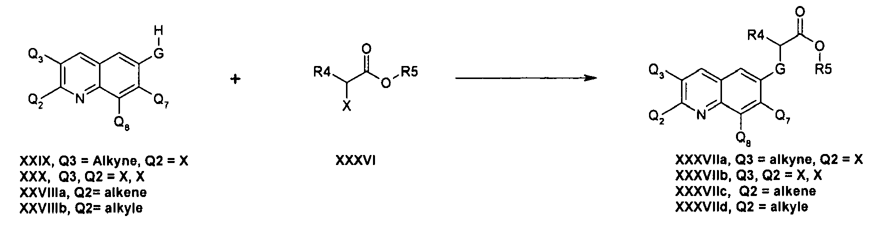

### Step XXVII + XXXIV → XXVIII

The step XXVII + XXXIV → XXVIII can be prepared in analogy to known procedures of the literature (Ma et al. J. Org. Chem. 2003, 68, 8726-8729; Umemoto et al. Chem. Res. Cent., J. Am. Chem. Soc. 1993, 115, 2156-2164; Yang et al. Synlett 1997, 2, 1379-1380; Bretschneider,H. et al. Monatshefte fuer Chemie 1965, 96, 1661 - 1676; Bendich J. Am. Chem. Soc.1953, 75, 4075; Luthman et al. J. Org. Chem. 1987, 52, 3777-3784).

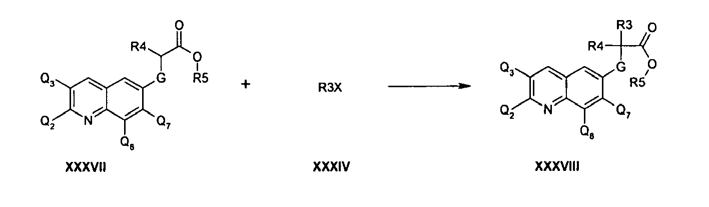

Inert solvents for the step XXVI + XXX → XXVI are for example ether like diethyl ether, dioxane, tetrahydrofurane, glycoldimethylether or diethylenglycoldimethylether or other solvent toluene, hexane, cyclohexane. It is also possible to use a mixture of these solvents. Tetrahydrofurane is preferred.

As bases for the step XXVI + XXX → XXVI are for example bases like n-butyllithium, sec-butyllithium, tert-butyllithium, lithium diisopropylamide, lithium hexamethyldisilazane, potassium hexamethyldisilazane.

### Step XXXVIIII + XIX → X

The ester is hydrolysed under usual methods, where the ester is treated with bases in inert solvents, the generated salts are then treated with acids to deliver the free carboxylic acids.

Water or the usual organic solvents for ester cleavage are suitable as inert solvents. These inert solvents are alcohols like methanol, ethanoln, propanol, isopropanol, n-butanol or tert-butanol ether like diethylether, tetrahydrofurane, dioxane or glycoldimethylether or other solvent like acetone, acetonitrile, dimethylformamide, dimethylsulfoxide. It is also possible to use a mixture of these solvents. In the case of a basic hydrolysis of esters, mixtures of water with dioxane, tetrahydrofurane, methanol and/or ethanol are preferred.

As bases are the usual inorganic bases suitable for the ester hydrolysis. These preferably include alkali hydroxide like sodium hydroxide, potassium hydroxide, lithium hydroxide or barium hydroxide or alkali carbonate like sodium carbonate, potassium carbonate, calcium carbonate or caesium carbonate. Sodium hydroxide or lithium hydroxide is preferred.

The ester hydrolysis is carried out at a temperature from 0°C to +100°C, preferentially from 0°C to +40°C. The reaction can be carried out at normal, high or low pressure (for example from 0.5 to 5 Bar). In general the reaction is carried out at normal pressure.

The carboxylic acid is then coupled with an amine. The coupling agent for the amide formation can be for example carbodiimide like N,N'-diethyl, N,N'-dipropyl, N,N'-diisopropyl, N,N'-dicyclohexylcarbodiimide (DCC), N-[3-(dimethylamino)propyl]-N'-ethylcarbodiimide hydrochloride (EDC), or N,N'-carbonyldiimidazol, or 1,2-oxazolium compounds like 2-ethyl-5-phenyl-1,2-oxazolium-3-sulfate or 2-tert.-butyl-5-methyl-isoxazolium-perchlorate or acylamino compounds like 2-ethoxy-1-ethoxycarbonyl-1,2-dihydrochinolin, or isobutylchloroformiate, 2,4,6-tripropyl-1,3,5,2,4,6-trioxatriphosphinane 2,4,6-trioxide, diethyl phosphorocyanidoate, bis-(2-oxo-3-oxazolidinyl)-phosphoryl chloride, (1H-benzotriazol-1-yloxy)[tris(dimethylamino)]phosphonium hexafluorophosphate, (1H-benzotriazol-1-yloxy)(tripyrrolidin-1-yl)phosphonium hexafluorophosphate (PyBOP), N-[(1H-benzotriazol-1-yloxy)(dimethylamino)methylidene]-N-methylmethanaminium tetrafluoroborate (TBTU), N-[(1H-benzotriazol-1-yloxy)(dimethylamino)methylidene]-N-methylmethanaminium hexafluorophosphate (HBTU), N-{(dimethylamino)[(2-oxopyridin-1(2H)-yl)oxy]methylidene}-N-methylmethanaminium tetrafluoroborate (TPTU), N-[(dimethylamino)(3H-[1,2,3]triazolo[4,5-b]pyridin-3-yloxy)methylidene]-N-methylmethanaminium hexafluorophosphate (HATU) or 1-[bis(dimethylamino)methylidene]-5-chloro-1H-benzotriazol-1-ium 3-oxide tetrafluoroborate (TCTU), if needed in combination with other derivative like 1H-benzotriazol-1-ol (HOBt) or N-hydroxysuccinimide (HOSu), as well as bases alkali carbonates, for example sodium or potassium carbonate, or sodium or potassium hydrogenocarbonate, or organic bases like trialkylamine, N-methylmorpholine, N-methylpiperidine or N,N-diisopropylethylamine.

This step is carried out in general in a temperature from -20°C to +60°C, preferentially 0°C to +40°C. The reaction can be carried out at normal, high or low pressure (for example from 0.5 to 5 Bar). In general the reaction is carried out at normal pressure.

Amides can be directly prepared in analogy to known procedures in the literature from esters and amines catalysed by a Lewis acids (MgBr2, MgCl2, Z. Guo et all Tetrahedron Letters, 2001, 42, 1843; AIMe3, Woodward et al Tetrahedron Letters 2006, 47, 5767, Tetrahedron Letters 2008 49, 5687) under reflux or micro wave irradiation (S. Woodward et al Tetrahedron Letters 2008 49, 5687).

Amides are directly prepared by treating esters with amines in presence of trialkylaluminium in an inert solvent.

The trialkylaluminium can be for example triethylaluminium, triisobutylaluminium, trimethylaluminium, tripropylaluminium, tri-tert-butylaluminum, tributylaluminum. Trimethylaluminium is preferred.

The organic solvents for direct amidation are ether like diethylether, tetrahydrofurane, dioxane or glycoldimethylether or other solvent like toluene. Tetrahydrofurane and toluene are preferred solvents. This step is carried out in general in a temperature from 0°C to boiling point, preferentially boiling point. The reaction can be carried out at normal, high or low pressure (for example from 0.5 to 5 Bar). In general the reaction is carried out at normal pressure.

The amidation can also be carried out under micro wave irradiation.

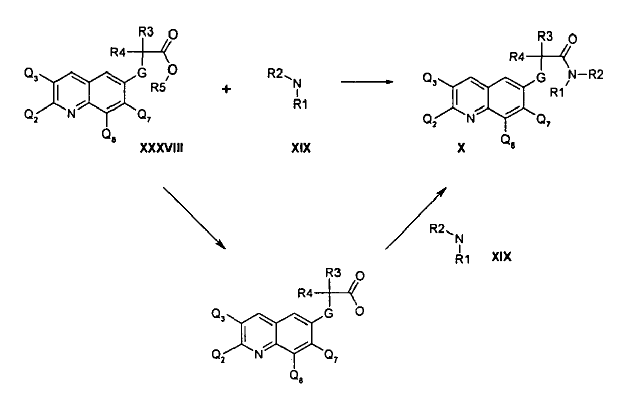

### Step X → XI

The thioamide XI is prepared by treating the corresponding amide with thionation reagent in acetonitrile, tetrahydrofurane, diglyme, toluene.

The thionation reagent can be for example phosphorus pentasulfide, 2,4-bis(4-methoxyphenyl)-1,3,2,4-dithiadiphosphetane 2,4-disulfite (Lawesson's reagent).

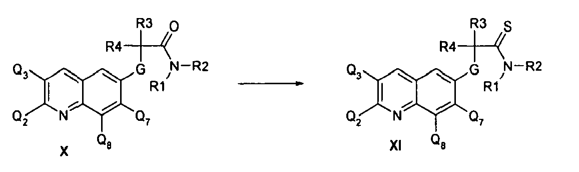

### Step XI + XXXX → XXXXIV

The compounds XXXXIV are prepared out in analogy to procedures described in the literature by treating the thioamides XI with a base followed by an alkyl halide (Bartsch et al Monatshefte fuer Chemie 1988, 119, 1439-1444; Bernath et al Tetrahedron 1987, 43, 4359-4366; O'Gorman et al Tetrahedron Letters 2008, 49, 6316-6319; Takahata et al Heterocycles 1985, 23, 2219-2215; Takahata et al J. Org. Chem 1990, 55, 3792-3797).

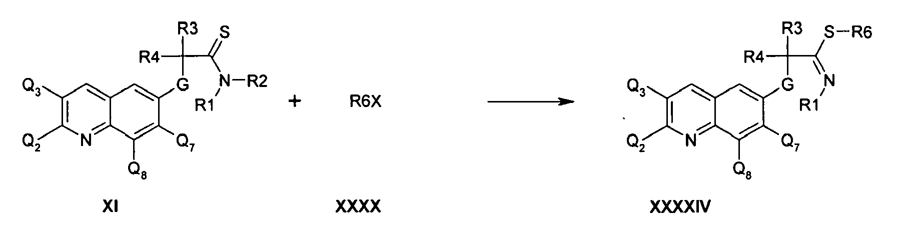

## Claims

1. A compound of formula (I) 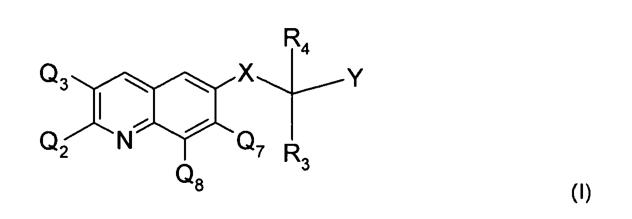 wherein
• Y is represented by 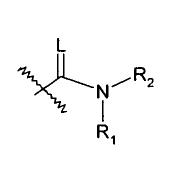 or 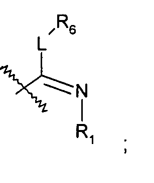
• X is O or S;
• LisOorS,
• Q2 is hydrogen, halogen, cyano, substituted or non-substituted, branched or non-branched C₁-C₈-alkyl, substituted or non-substituted C₃-C₆-cydoalkyl, C₃-C₁₂-(cycloalkyl)alkyl, substituted or non-substituted C₂-C₆-alkenyl, or substituted or non-substituted C₂-C₆-alkynyl,
• Q3 is halogen, or substituted or non-substituted C₂-C₆-alkynyl,
• Q7 is hydrogen, halogen, cyano, substituted or non-substituted, branched or non-branched C₁-C₈-alkyl, substituted or non-substituted C₁-C₆-haloalkyl, substituted or non-substituted C₃-C₆-cydoalkyl, C₃-C₁₂-(cycloalkyl)alkyl, substituted or non-substituted C₁-C₆-alkoxy, or alkylsulfinyl,
• Q8 is hydrogen, halogen, cyano, substituted or non-substituted, branched or non-branched C₁-C₈-alkyl, substituted or non-substituted C₁-C₆-haloalkyl, substituted or non-substituted C₃-C₆-cycloalkyl, C₃-C₁₂-(cycloalkyl)alkyl, substituted or non-substituted C₁-C₆-alkoxy, or alkylsulfinyl,
• R3 is halogen, cyano, substituted or non-substituted, branched or non-branched C₁-C₈-alkyl, substituted or non-substituted C₁-C₆-haloalkyl, substituted or non-substituted C₃-C₆-cycloalkyl, C₁-C₈-alkoxycarbonyl, or a heteroaryl non-substituted or substituted with substituted or non-substituted, branched or non-branched alkyl C₁-C₆ or cycloclalkyl C₃-C₆, wherein the heteroaryl is a 5-membered ring having one to three nitrogen atom(s) and is bound by a nitrogen atom, with the proviso that, when R4 is hydrogen, then R3 is not a C₁-C₈-alkyl,
• R4 is hydrogen, substituted or non-substituted, branched or non-branched C₁-C₈-alkyl, substituted or non-substituted C₁-C₈-alkoxy, C₁-C₄-alkylsulfinyl, S(O)nR7,
• R2 is hydrogen, substituted or non-substituted, branched or non-branched C₁-C₈-alkyl, substituted or non-substituted C₃-C₄-cycloalkyl, or substituted or non-substituted C₁-C₄-cyanoalkyl,
• R1 is heteroaryl, heteroarylalkyl, C₁-C₈-alkyloxyalkyl, C₃-C₅-cycloalkyl, C₁-C₈-alkyl, Alkynylalkyl, Alkylalkynylalkyl, Alkylalkynylcycloalkyl, or Alkoxylalkylalkynylalkyl,
• R6 is substituted or non-substituted, branched or non-branched C₁-C₈-alkyl, substituted or non-substituted C₁-C₆-haloalkyl, or substituted or non-substituted C₃-C₆-cycloalkyl,
• R7 is substituted or non-substituted, branched or non-branched C₁-C₈-alkyl,
• n is 0, 1 or 2;
• unless indicated otherwise, a group or a substituent that is substituted is substituted by one or more of the following groups or atoms: a halogen atom, a nitro group, a hydroxy group, a cyano group, an amino group, a sulphenyl group, a pentafluoro-λ⁶-sulphenyl group, a formyl group, a substituted or non-substituted carbaldehyde O-(C₁-C₈-alkyl)oxime, a formyloxy group, a formylamino group, a carbamoyl group, a N-hydroxycarbamoyl group, a (hydroxyimino)-C₁-C₆-alkyl group, a C₁-C₈-alkyl, a tri(C₁-C₈-alkyl)silyl-C₁-C₈-alkyl, C₁-C₈-cycloalkyl, tri(C₁-C₈-alkyl)silyl-C₁-C₈-cycloalkyl, a C₁-C₈-halogenoalkyl having 1 to 5 halogen atoms, a C₁-C₈-halogenocycloalkyl having 1 to 5 halogen atoms, a C₂-C₈-alkenyl, a C₂-C₈-alkynyl, a C₂-C₈-alkenyloxy, a C₂-C₈-alkynyloxy, a C₁-C₈-alkylamino, a di-C₁-C₈-alkylamino, a C₁-C₈-alkoxy, a C₁-C₈-halogenoalkoxy having 1 to 5 halogen atoms, a C₁-C₈-alkylsulphenyl, a C₁-C₈-halogenoalkylsulphenyl having 1 to 5 halogen atoms, a C₂-C₈-alkenyloxy, a C₂-C₈-halogenoalkenyloxy having 1 to 5 halogen atoms, a C₃-C₈-alkynyloxy, a C₃-C₈-halogenoalkynyloxy having 1 to 5 halogen atoms, a C₁-C₈-alkylcarbonyl, a C₁-C₈-halogenoalkylcarbonyl having 1 to 5 halogen atoms, a C₁-C₈-alkylcarbamoyl, a di-C₁-C₈-alkylcarbamoyl, a N-C₁-C₈-alkyloxycarbamoyl, a C₁-C₈-alkoxycarbamoyl, a N-C₁-C₈-alkyl-C₁-C₈-alkoxycarbamoyl, a C₁-C₈-alkoxycarbonyl, a C₁-C₈-halogenoalkoxycarbonyl having 1 to 5 halogen atoms, a C₁-C₈-alkylcarbonyloxy, a C₁-C₈-halogenoalkylcarbonyloxy having 1 to 5 halogen atoms, a C₁-C₈-alkylcarbonylamino, a C₁-C₈-halogenoalkylcarbonylamino having 1 to 5 halogen atoms, substituted or non-substituted C₁-C₈-alkoxycarbonylamino, substituted or non-substituted C₁-C₈-halogenoalkoxycarbonylamino having 1 to 5 halogen atoms, a C₁-C₈-alkylaminocarbonyloxy, a di-C₁-C₈-alkylaminocarbonyloxy, a C₁-C₈-alkyloxycarbonyloxy, a C₁-C₈-alkylsulphenyl, a C₁-C₈-halogenoalkylsulphenyl having 1 to 5 halogen atoms, a C₁-C₈-alkylsulphinyl, a C₁-C₈-halogenoalkylsulphinyl having 1 to 5 halogen atoms, a C₁-C₈-alkylsulphonyl, a C₁-C₈-halogenoalkylsulphonyl having 1 to 5 halogen atoms, a C₁-C₈-alkylaminosulfamoyl, a di-C₁-C₈-alkylaminosulfamoyl, a (C₁-C₆-alkoxyimino)-C₁-C₆-alkyl, a (C₁-C₆-alkenyloxyimino)-C₁-C₆-alkyl, a (C₁-C₆-alkynyloxyimino)-C₁-C₆-alkyl, (benzyloxyimino)-C₁-C₆-alkyl, C₁-C₈-alkoxyalkyl, C₁-C₈-halogenoalkoxyalkyl having 1 to 5 halogen atoms, benzyloxy, benzylsulphenyl, benzylamino, phenoxy, phenylsulphenyl, or phenylamino.

2. A fungicide composition comprising, as an active ingredient, an effective amount of a compound of formula (I) according to claim 1 and an agriculturally acceptable support, carrier or filler.

3. A method for controlling phytopathogenic fungi of plants, crops or seeds, **characterized in that** an agronomically effective and substantially non-phytotoxic quantity of a compound according to claim 1 or a composition according to claim 2 is applied to the soil where plants grow or are capable of growing, to the leaves and/or the fruit of plants or to the seeds of plants.
